# EUROPEAN PATENT APPLICATION

(11) **EP 2 518 072 A1**
(43) Date of publication of application: **31.10.2012**
(21) Application number: 10839451.1
(22) Date of filing: 22.12.2010
(51) Int. Cl.: C07D 487/04, A61K 31/5377, A61P 1/04, A61P 1/16, A61P 3/10, A61P 7/04, A61P 7/06, A61P 9/00, A61P 11/00, A61P 13/12, A61P 17/00, A61P 17/04

(54) **IMIDAZOPYRIDAZINE COMPOUNDS**

(30) Priority: 24.12.2009 JP 2009293145; 28.06.2010 JP 2010146436
(71) Applicant: AJINOMOTO CO., INC., Chuo-ku Tokyo 104-8315 (JP)
(72) Inventor: YAMAMOTO Takashi, Kawasaki-shi Kanagawa 210-0801 (JP); ANDO Ayatoshi, Kawasaki-shi Kanagawa 210-0801 (JP); HAYAKAWA Nobuhiko, Kawasaki-shi Kanagawa 210-0801 (JP); NOGUCHI Masatsugu, Kawasaki-shi Kanagawa 210-0801 (JP); NISHIO Hikaru, Kawasaki-shi Kanagawa 210-0801 (JP); EVIRYANTI Agung, Kawasaki-shi Kanagawa 210-0801 (JP); YOKOYAMA Ryohei, Kawasaki-shi Kanagawa 210-0801 (JP); KAGEYAMA Shunsuke, Kawasaki-shi Kanagawa 210-0801 (JP); KURIBAYASHI Kanna, Kawasaki-shi Kanagawa 210-8681 (JP)
(74) Representative: Nicholls, Kathryn Margaret
(86) International application number: PCT/JP2010/073126
(87) International publication number: WO 2011/078221

(57) **Abstract**

By searching various compounds having an inhibiting activity against IL-12/IL-23 production, the present invention provides a pharmaceutical composition or a therapeutic or preventive agent for diseases related to IL-12/IL-23 excessive production each of which comprises the compound(s) of the present invention.

More specifically, the present invention provides an imidazopyridazine compound having an inhibiting activity against IL-12/IL-23 production or pharmaceutically acceptable salts thereof; and a useful pharmaceutical composition or a useful therapeutic or preventive agent for diseases related to IL-12/IL-23 excessive production each of which comprises said compound or pharmaceutically acceptable salts thereof.

## Description

### Technical Field of the Invention

The present invention relates to novel imidazopyridazine compounds having an inhibiting activity against IL-12/IL-23 production or pharmaceutically acceptable salts thereof; and pharmaceutical compositions or therapeutic or preventive agents for diseases related to IL-12/IL-23 excessive production each of which comprise the imidazopyridazine compound or pharmaceutically acceptable salts thereof as an active ingredient.

Meanwhile, inhibition against IL-12/IL-23 production means the inhibition against IL-12 production, the inhibition against IL-23, or the inhibition against IL-12 and IL-23 production. Similarly, IL-12/IL-23 excessive production means the excessive production of IL-12, the excessive production of IL-23, or the excessive production of IL-12 and IL-23.

### Background of the Invention

Interleukin-12 (IL-12) is a heterodimeric inflammatory cytokine consisting of two subunits, p35 and p40 (IL-12 is also referred to as IL-12p70). IL-12 plays an important role in the immune response by acting as a bridge between congenital resistance and antigen-specific adaptive immunity. Due to the stimulation by bacteria, bacterial products such as lipopolysaccharide (LPS) and intracellular parasite, IL-12 is produced by phagocytes and antigen presenting cells, and particularly by macrophage and dendritic cells. Well known biological functions of IL-12 is the induction of expression of interferon- γ (IFN- γ) from T cells and NK cells and the induction of differentiating T cells into Th1 T lymphocyte type. Interleukin-23 (IL-23) is, as well as IL-12, a heterodimeric inflammatory cytokine consisting of two subunits, p19 and p40. IL-23 is involved in type I immune defense and induces the secretion of IFN- γ from T cells.

As mentioned above, both IL-12 and IL-23 have p40 subunit and play an important role in the immune inflammatory response. On the other hand, in chronic diseases associated with the continuous production of IFN- γ, it has been indicated that IL-12 production is enhanced by IFN- γ. Therefore, it is thought that, after infectious stimulation or inflammatory stimulation each of which induces IL-12 production, an extremely strong feedback loop is formed wherein IL-12-induced and IL-23-induced IFN-γ further promotes IL-12 production, and thus, excessive production of proinflammatory cytokine is induced.

IL-12/IL-23 excessive production is involved in various diseases, e.g., multiple sclerosis, systemic sclerosis, sepsis, myasthenia gravis, autoimmune neurological disease, Guillain-Barre syndrome, autoimmune uveitides, autoimmune hemolytic anemia, pernicious anemia, autoimmune thrombocytopenia, temporal arteritis, antiphospholipid syndrome, vasculitis, Wegener's granulomatosis, Behcet's disease, psoriasis, psoriatic arthritis, herpetic dermatitis, pemphigus vulgaris, vitiligo, Crohn's disease, ulcerative colitis, interstitial fibroid lung, myelofibrosis, hepatic fibrosis, myocarditis, autoimmune thyroid disease (Graves' disease, Hashimoto's disease), primary biliary cirrhosis, autoimmune hepatitis, immune-mediated diabetes mellitus, autoimmune oophoritis and orchitis, autoimmune adrenalitis, rheumatoid arthritis, juvenile rheumatoid arthritis, systemic lupus erythematosus, scleroderma, polymyositis, dermatomyositis, spondyloarthropathy, ankylosing spondylitis, Sjögren's syndrome, graft versus host disease, and ischemic vascular disorder, though it is not limited to these diseases.

On the other hand, same as IL-12/IL-23, TNF- α is one of the inflammatory cytokines playing a central role in developing the condition of rheumatoid arthritis or the like. Actually, in clinical practice in Japan, biological preparations targeting TNF- α are used such as etanercept which is a fusion protein of sTNFR and immunoglobulin G, and infliximab and adalimumab each of which is an anti-TNF- α monoclonal antibody. However, it is thought that TNF- α is a cytokine extremely important in the immune response and located upstream of the immune response signals. Therefore, the above preparations have many side effects, and there are concerns that they have a high risk of developing infection, cancer, or the like.

Accordingly, a compound inhibiting IL-12/IL-23 production can have a high selectivity against diseases related to IL-12/IL-23 excessive production with less side effects, and can be a clinically extremely useful therapeutic or preventive agent for various inflammatory diseases including the above diseases.

Each of Patent Literatures 1-9 discloses a compound having an inhibiting activity against IL-12 excessive production, but the compound disclosed therein has a different skeleton from an imidazo[1,2-b]pyridazine skeleton of the present invention. Further, each of Japanese patent application No. 2008-334965 and Japanese patent application No. 2009-228755 which is a priority application of 2008-334965 discloses a compound having an inhibiting activity against IL-12 excessive production, but the compound disclosed therein has a pyrazolo[1,5-b]pyrimidine skeleton, which is different from an imidazo[1,2-b]pyridazine skeleton that the compound of the present invention has.

Each of Patent Literatures 10 and 11 discloses a condensed heterocyclic compound having an inhibiting activity against MAPKAP kinase-2 (MK2), but it is not disclosed that the compound disclosed therein has an inhibiting activity against IL-12/IL-23 production that the compound of the present invention has. Patent Literature 12 discloses a condensed heterocyclic compound having an inhibiting activity against protein kinase CK2, but it is not disclosed that the compound disclosed therein has an inhibiting activity against IL-12/IL-23 production that the compound of the present invention has.

Accordingly, it is demanded to provide a clinically extremely useful therapeutic or preventive agent for various inflammatory diseases including the above diseases, and said agent having an inhibiting activity against IL-12/IL-23 production, having a high selectivity against diseases related to IL-12/IL-23 excessive production with less side effects.
Patent Literature 1: WO 2003/047516 A2
Patent Literature 2: WO 2005/046698 A1
Patent Literature 3: WO 2004/035740 A2
Patent Literature 4: WO 2005/046603 A2
Patent Literature 5: WO 2005/046604 A2
Patent Literature 6: WO 2006/007532 A2
Patent Literature 7: WO 2006/053109 A1
Patent Literature 8: WO 2006/053227 A2
Patent Literature 9: WO 2006/124662 A1
Patent Literature 10: US 2008/0045536 A1
Patent Literature 11: WO 2007/038314 A2
Patent Literature 12: WO 2009/100375 A1

### Disclosure of the Invention

The object of the present invention is, by searching various variation compounds having an inhibiting activity against IL-12/IL-23 production, to provide a pharmaceutical composition or a therapeutic or preventive agent for diseases related to IL-12/IL-23 excessive production each of which comprises said compound(s).

The inventors searched compounds having an inhibiting activity against IL-12/IL-23 production and found that, surprisingly, a specific novel imidazopyridazine compound or pharmaceutically acceptable salts thereof have an excellent inhibiting activity against IL-12/IL-23 production. The present invention has been completed based on this finding. The imidazopyridazine compound or pharmaceutically acceptable salts thereof can be a useful pharmaceutical composition or a useful therapeutic or preventive agent for diseases related to IL-12/IL-23 excessive production.

Namely, the present invention provides an imidazopyridazine compound of the following formula (I) or pharmaceutically acceptable salts thereof: 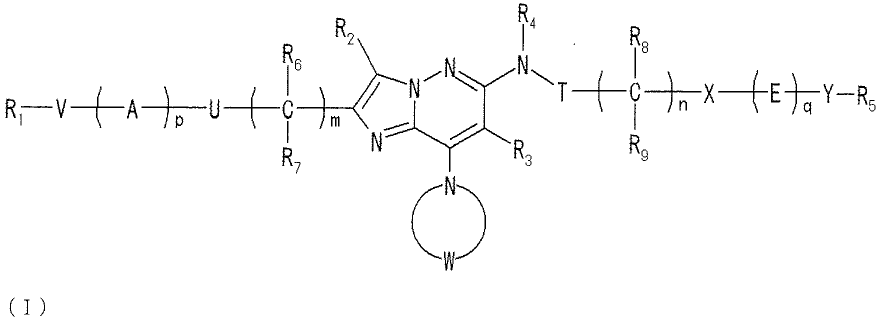
wherein A and E may be same or different from each other and each independently represents an aryl group which may have a substituent(s), a heterocyclic group which may have a substituent(s) or an aliphatic cyclic group which may have a substituent(s);
U, V, X and Y may be same or different from each other and each independently represents a single bond, O, S, S(O), S(O₂) NRa, C(O), C(O)O, C(O)NRa, OC(O), NRaC(O), NRaC(O)NRb, OC(O)NRa, NRaC(O)O, S(O)NRa, S(O₂)NRa, NRaS(O), NRaS(O₂), CRa=CRb, C≡C or CRa=N wherein Ra and Rb each independently represents a hydrogen atom or a straight or branched alkyl group having 1 to 3 carbon atoms;
T represents NRcC(RdRe) or N=C(Rd) wherein Rc, Rd and Re each independently represents a hydrogen atom or a straight or branched alkyl group having 1 to 3 carbon atoms;
m and n may be same or different from each other and each independently represents 0, 1 or 2;
p and q may be same or different from each other and each independently represents 0 or 1;
R₁ represents a hydrogen atom, a halogeno group, a hydroxyl group, an alkyl group which may have a substituent(s), a mercapto group, an alkoxy group which may have a substituent(s), an alkylthio group which may have a substituent(s), an alkylsulfonyl group which may have a substituent(s), an acyl group which may have a substituent(s), an acyloxy group which may have a substituent(s), an amino group which may have a substituent(s), a carboxyl group, an alkoxycarbonyl group which may have a substituent(s), a carbamoyl group which may have a substituent(s), a nitro group, a cyano group, a trifluoromethyl group, a sulfonic group, a sulfonamide group which may have a substituent(s), a sulfinamide group which may have a substituent(s), an aliphatic cyclic group which may have a substituent(s), an alkenyl group which may have a substituent(s), an alkynyl group which may have a substituent(s), a benzyloxy group which may have a substituent(s), an aryloxy group which may have a substituent(s), an arylvinyl group which may have a substituent(s), a heteroaryloxy group which may have a substituent(s), an arylamino group which may have a substituent(s), a heteroarylamino group which may have a substituent(s), an arylethynyl group which may have a substituent(s), an aralkyl group which may have a substituent(s), an aryl group which may have a substituent(s) or a heterocyclic group which may have a substituent(s);
R₂ represents a hydrogen atom, a halogeno group or a straight or branched alkyl group having 1 to 3 carbon atoms; R₃ represents a hydrogen atom or a straight or branched alkyl group having 1 to 3 carbon atoms;
R₄ represents a hydrogen atom or a straight or branched alkyl group having 1 to 3 carbon atoms;
R₅ represents a hydrogen atom, a halogeno group, a hydroxyl group, a boronyl group which may have a substituent(s), an alkyl group which may have a substituent(s), a mercapto group, an alkoxy group which may have a substituent(s), an alkylthio group which may have a substituent(s), an alkylsulfonyl group which may have a substituent(s), an acyl group which may have a substituent(s), an acyloxy group which may have a substituent(s), an amino group which may have a substituent(s), a carboxyl group, an alkoxycarbonyl group which may have a substituent(s), a carbamoyl group which may have a substituent(s), a nitro group, a cyano group, a trifluoromethyl group, a sulfonic group, a sulfonamide group which may have a substituent(s), a sulfinamide group which may have a substituent(s), an aliphatic cyclic group which may have a substituent(s), an alkenyl group which may have a substituent(s), an alkynyl group which may have a substituent(s), a benzyloxy group which may have a substituent(s), an aryloxy group which may have a substituent(s), an arylvinyl group which may have a substituent(s), a heteroaryloxy group which may have a substituent(s), an arylamino group which may have a substituent(s), a heteroarylamino group which may have a substituent(s), an arylethynyl group which may have a substituent(s), an aralkyl group which may have a substituent(s), an aryl group which may have a substituent(s) or a heterocyclic group which may have a substituent(s);
R₆, R₇, R₈ and R₉ may be same or different from each other and each independently represents a hydrogen atom, a halogeno group or a straight or branched alkyl group having 1 to 3 carbon atoms; and
the group of the following (II) having W represents a four- to six-membered cyclic amino group which may have a substituent(s), wherein W represents O, S or NRf (Rf represents a hydrogen atom or a straight or branched alkyl group having 1 to 3 carbon atoms):

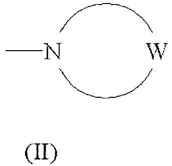

The present invention also provides a pharmaceutical composition or a therapeutic or preventive agent for diseases related to IL-12/IL-23 excessive production each of which comprises the imidazopyridazine compound of the above formula (I) or pharmaceutically acceptable salts thereof.

The compound of the present invention selectively inhibits IL-12/IL-23 production and, preferably, does not significantly inhibit TNF- α production from activated macrophage. Thus, the compound of the present invention has a high selectivity as a therapeutic or preventive agent for diseases related to IL-12/IL-23 excessive production and does not bring concern about side effects associated with the compounds having an inhibiting activity against TNF- α production. Further, in the case of the preferable compound of the present invention, the inhibiting activity thereof against IL-12/IL-23 production is not significantly reduced in whole blood, and thus, an extremely excellent medicinal effect can be provided in the administration to a human being in clinical practice.

### Best Mode for Carrying out the Invention

In the present invention, an aryl group represents a mono- or bi-cyclic aromatic substituent having 5 to 12 carbon atoms. Examples thereof include a phenyl group, an indenyl group, a naphthyl group and a fluorenyl group, and a phenyl group is preferable among them.

An aralkyl group represents a lower alkyl group substituted with an aryl group, wherein the aryl group represents the above aryl group. Examples thereof include a benzyl group and a phenethyl group.

Examples of a halogeno group include a fluorine atom, a chlorine atom, a bromine atom and an iodine atom.

An alkyl group represents a straight or branched or cyclic alkyl group having 1 to 18 carbon atoms. Examples thereof include a methyl group, ethyl group, n-propyl group, n-butyl group, n-pentyl group, n-hexyl group, n-heptyl group, n-octyl group, n-nonyl group, n-decyl group, n-undecyl group, n-dodecyl group, isopropyl group, isobutyl group, sec-butyl group, tert-butyl group, isopentyl group, tert-pentyl group, neopentyl group, 2-pentyl group, 3-pentyl group, 3-hexyl group, 2-hexyl group, tert-octyl group, cyclopropyl group, cyclobutyl group, cyclopentyl group, cyclohexyl group and 1-adamantyl group. An n-hexyl group, n-heptyl group, n-octyl group, n-nonyl group, n-decyl group, n-undecyl group, n-dodecyl group, isopropyl group, isobutyl group, sec-butyl group, tert-butyl group, isopentyl group, tert-pentyl group, neopentyl group, 2-pentyl group, 3-pentyl group, 3-hexyl group, 2-hexyl group, tert-octyl group, cyclopropyl group, cyclobutyl group, cyclopentyl group, cyclohexyl group and 1-adamantyl group are preferable among them, and an isopropyl group, tert-butyl group, tert-octyl group and 1-adamantyl group are more preferable. A lower alkyl group represents a straight or branched or cyclic alkyl group having 1 to 6 carbon atoms and preferably having 1 to 3 carbon atoms. Examples thereof include a methyl group, ethyl group, n-propyl group, n-butyl group, n-pentyl group, n-hexyl group, isopropyl group, isobutyl group, sec-butyl group, tert-butyl group, isopentyl group, tert-pentyl group, neopentyl group, 2-pentyl group, 3-pentyl group, 3-hexyl group, 2-hexyl group, cyclopropyl group, cyclobutyl group, cyclopentyl group and cyclohexyl group. A methyl group and an ethyl group are preferable among them.

An alkenyl group represents a straight or branched or cyclic alkenyl group having 1 to 6 carbon atoms. Examples thereof include a vinyl group, 1-propenyl group, 2-propenyl group, isopropenyl group, 1-butenyl group, 2-butenyl group and 3-butenyl group.

An alkynyl group represents a straight or branched alkynyl group having 1 to 6 carbon atoms. Examples thereof include an ethynyl group, 1-propynyl group, 2-propynyl group, 1-butynyl group, 2-butynyl group and 3-butynyl group.

An alkoxy group represents an alkoxy group which has a straight or branched or cyclic alkyl group having 1 to 18 carbon atoms and preferably having 1 to 8 carbon atoms. Examples thereof include a methoxy group, ethoxy group, n-propoxy group, n-butoxy group, n-pentyloxy group, n-hexyloxy group, n-heptyloxy group, n-octyloxy group, n-nonyloxy group, n-decyloxy group, n-undecyloxy group, n-dodecyloxy group, isopropoxy group, isobutoxy group, sec-butoxy group, tert-butoxy group, cyclopropyloxy group, cyclobutoxy group, cyclopentyloxy group, cyclohexyloxy group, cycloheptyloxy group, 2-cyclohexylethoxy group, 1-adamantyloxy group, 2-adamantyloxy group, 1-adamantylmethyloxy group, 2-(1-adamantyl)ethyloxy group and trifluoromethoxy group. A methoxy group, ethoxy group, n-propoxy group, isopropoxy group, n-butoxy group, tert-butoxy group, n-pentyloxy group and n-hexyloxy group are preferable among them.

An alkylthio group represents an alkylthio group which has a straight or branched or cyclic alkyl group having 1 to 12 carbon atoms and preferably having 1 to 6 carbon atoms. Examples thereof include a methylthio group, ethylthio group, n-propylthio group, isopropylthio group, n-butylthio group, isobutylthio group, sec-butylthio group, tert-butylthio group, cyclopropylthio group, cyclobutylthio group, cyclopentylthio group and cyclobutylthio group.

An alkylsulfonyl group represents an alkylsulfonyl group which has a straight or branched or cyclic alkyl group having 1 to 12 carbon atoms. Examples thereof include a methanesulfonyl group, ethanesulfonyl group, propanesulfonyl group, butanesulfonyl group, pentanesulfonyl group, hexanesulfonyl group, heptanesulfonyl group, octanesulfonyl group, nonanesulfonyl group, decanesulfonyl group, undecanesulfonyl group and dodecanesulfonyl group.

An acyl group represents a formyl group, an acyl group which has a straight or branched or cyclic alkyl group having 1 to 6 carbon atoms, an acyl group which has a straight or branched or cyclic alkenyl group having 1 to 6 carbon atoms, an acyl group which has a straight or branched or cyclic alkynyl group having 1 to 6 carbon atoms, or an acyl group which has an aryl group that may be substituted. Examples thereof include a formyl group, acetyl group, propionyl group, butyryl group, isobutyryl group, valeryl group, isovaleryl group, pivaloyl group, hexanoyl group, acryloyl group, metacryloyl group, crotonoyl group, isocrotonoyl group, benzoyl group and naphthoyl group.

An acyloxy group represents a formyloxy group, an acyloxy group which has a straight or branched or cyclic alkyl group having 1 to 6 carbon atoms, or an acyloxy group which has an aryl group that may be substituted. Examples thereof include a formyloxy group, acetyloxy group, propionyloxy group, butyryloxy group, isobutyryloxy group, valeryloxy group, isovaleryloxy group, pivaloyloxy group, hexanoyloxy group, acryloyloxy group, metacryloyloxy group, crotonoyloxy group, isocrotonoyloxy group, benzoyloxy group and naphthoyloxy group.

An alkoxycarbonyl group represents an alkoxycarbonyl group which has a straight or branched or cyclic alkyl group having 1 to 8 carbon atoms. Examples thereof include a methoxycarbonyl group, ethoxycarbonyl group, propoxycarbonyl group, isopropoxycarbonyl group, n-butoxycarbonyl group, isobutoxycarbonyl group, sec-butoxycarbonyl group, tert-butoxycarbonyl group and benzyloxycarbonyl group.

A carbamoyl group represents a carbamoyl group which may have a straight or branched or cyclic alkyl group having 1 to 6 carbon atoms on nitrogen. Examples thereof include a carbamoyl group, N-methylcarbamoyl group, N-ethylcarbamoyl group, N,N-dimethylcarbamoyl group, N-pyrrolidylcarbonyl group, N-piperidylcarbonyl group and N-morpholinylcarbonyl group.

A heterocyclic group represents a heterocyclic group consisting of one, two or three five- to seven-membered ring(s) composed of carbon and nitrogen, oxygen, sulfur and the like. Examples thereof include a pyridine ring, dihydropyran ring, pyridazine ring, pyrimidine ring, pyrazine ring, pyrrole ring, furan ring, thiophene ring, oxazole ring, isoxazole ring, pyrazole ring, imidazole ring, thiazole ring, isothiazole ring, thiadiazole ring, pyrrolidine ring, piperidine ring, piperazine ring, indole ring, isoindole ring, benzofuran ring, isobenzofuran ring, benzothiophene ring, benzopyrazole ring, benzoimidazole ring, benzoxazole ring, benzothiazole ring, purine ring, pyrazolopyridine ring, quinoline ring, isoquinoline ring, naphthyridine ring, quinazoline ring, benzodiazepine ring, carbazole ring, dibenzofuran ring, thiazolidine ring, morpholine ring, imidazothiazole ring and dihydrobenzofuran ring. A pyridine ring, pyrimidine ring, pyridazine ring, furan ring and thiophene ring are preferable among them, and a pyridine ring, pyrimidine ring and thiophene ring are more preferable.

An aliphatic cyclic group represents a monocyclic or bicyclic aliphatic group which is composed of a carbon atom(s). Examples thereof include a cyclopropane ring, cyclobutane ring, cyclopenane ring, cyclohexane ring, cycloheptane ring, cyclooctane ring, decalin ring and norbornane ring, and a cylohexane ring is preferable among them.

An aryloxy group represents an aryloxy group having an aryl group on an oxygen atom, and examples of the aryl group are the same as those mentioned in the above "aryl group." Examples of the aryloxy group include a phenoxy group, 1-naphthyloxy group and 2-naphthyloxy group.

An arylamino group represents an arylamino group having an aryl group on a nitrogen atom and examples of the aryl group are the same as those mentioned in the above "aryl group." Examples of the arylamino group include a phenylamino group, 1-naphthylamino group and 2-naphthylamino group.

An arylvinyl group represents a vinyl group of which the first position or the second position is substituted with an aryl group, and examples of the aryl group are the same as those mentioned in the above "aryl group." Examples of the arylvinyl group include a 1-phenylvinyl group and 2-phenylvinyl group.

An arylethynyl group represents an ethynyl group of which the second position is substituted with an aryl group, and examples of the aryl group are the same as those mentioned in the above "aryl group." Examples of the arylethynyl group include a phenylethynyl group.

A heteroaryl group represents a heteroaromatic substituent consisting of one, two or three five- to seven-membered ring(s) composed of carbon and nitrogen, oxygen, sulfur and the like. Examples thereof include a pyridyl group, pyridazinyl group, pyrimidinyl group, pyrazinyl group, pyrrolyl group, furyl group, thienyl group, oxazolyl group, isoxazolyl group, pyrazolyl group, imidazolyl group, thiazolyl group, isothiazolyl group, thiadiazolyl group, indolyl group, isoindolyl group, benzofuryl group, isobenzofuryl group, benzothiophenyl group, benzopyrazolyl group, benzoimidazolyl group, benzoxazolyl group, benzothiazolyl group, quinolyl group, isoquinolyl group, naphthyridinyl group and quinazolyl group. A 2-pyridyl group, 3-pyridyl group, 4-pyridyl group, 1-pyrazolyl group and 2-pyrazinyl group are preferable among them. Further, a 2-pyridyl group, 3-pyridyl group, 4-pyridyl group, 1-pyrazolyl group, 2-pyrazinyl group, 2-indolyl group, 3-indolyl group, 4-indolyl group, 5-indolyl group, 6-indolyl group, 3-pyrazolyl group and 4-pyrazolyl group are also preferable.

A heteroaryloxy group is a heteroaryloxy group having a heteroaryl group on an oxygen atom, and examples of the heteroaryl group are the same as those mentioned in the above "heteroaryl group." Examples of the heteroaryloxy group include a 2-pyridyloxy group, 3-pyridyloxy group, 4-pyridyloxy group and 2-pyrimidinyl group.

A heteroarylamino group is a heteroarylamino group having a heteroaryl group on a nitrogen atom and examples of the heteroaryl group are the same as those mentioned in the above "heteroaryl group." Examples of the heteroarylamino group include a 2-pyridylamino group, 3-pyridylamino group, 4-pyridylamino group and 2-pyrimidinylamino group.

The term "which may have a substituent(s)" indicates that a group may not have any substituent or a group may have one or more substituent(s). In the case that a group has a substituent(s), the group is substituted with at least one or more substituent(s). The substituent(s) may be same or different from each other, and the position and number thereof are preferably selected and not particularly limited, but the number of the substituent(s) is preferably 1, 2 or 3, and particularly preferably 1 or 2. Examples of the substituent include a halogeno group, hydroxyl group, lower alkyl group, mercapto group, alkoxy group having 1 to 6 carbon atoms, alkylthio group having 1 to 6 carbon atoms, lower alkylsulfonyl group, acyl group having 1 to 6 carbon atoms, acyloxy group having 1 to 6 carbon atoms, amino group, alkylamino group having 1 to 6 carbon atoms, carboxyl group, alkoxycarbonyl group having 2 to 6 carbon atoms, carbamoyl group, alkylcarbamoyl group having 2 to 6 carbon atoms, nitro group, cyano group, trifluoromethyl group, sulfonic group, sulfonamide group, sulfinamide group, aliphatic cyclic group having 1 to 6 carbon atoms, alkenyl group having 2 to 6 carbon atoms, alkynyl group having 2 to 6 carbon atoms, aryl group having 6 to 10 carbon atoms and heterocyclic group having 3 to 9 carbon atoms.

In the formula (I), A is preferably an aryl group which may have a substituent(s) or a heterocyclic group which may have a substituent(s). As the substituent(s), a halogeno group, a hydroxyl group and an alkyl group which may have a substituent(s) are preferable. A lower alkyl group is preferable among them, and a methyl group is particularly preferable. A is also preferably an aryl group without any substituent or, more preferably, a heterocyclic group without any substituent.

Preferable examples of A include a phenyl group, naphthyl group, pyridyl group, pyridazyl group, pyrimidinyl group, pyrazyl group, quinolyl group, isoquinolyl group, imidazolyl group, benzoimidazolyl group, pyrrolyl group, indolyl group, furyl group, benzofuryl group, thienyl group, benzothiophenyl group, oxazolyl group, benzoxazolyl group, isoxazolyl group, benzisoxazolyl group, thiazolyl group, benzothiazolyl group, isothiazolyl group, benzisothiazolyl group, pyrazolyl group, benzopyrazolyl group, imidazothiazolyl group, aziridino group, azetidino group, pyrrolidino group, piperidino group, piperazino group, methylpiperazino group, morpholinyl group, thiazolidino group and thiomorpholinyl group. Particularly, a phenyl group, pyridyl group, methylpyrazolyl group and thienyl group are preferable among them.

E is preferably an aryl group which may have a substituent(s) or a heterocyclic group which may have a substituent(s). As the substituent(s), a halogeno group, a hydroxyl group, an alkyl group which may have a substituent(s) and an alkenyl group which may have a substituent(s) are preferable. A lower alkyl group is preferable among them, and a methyl group is particularly preferable. E is also preferably an aryl group without any substituent or a heteroaryl group without any substituent.

Preferable examples of E include a phenyl group, naphthyl group, pyridyl group, pyridazyl group, pyrimidinyl group, pyrazyl group, quinolyl group, isoquinolyl group, imidazolyl group, benzoimidazolyl group, pyrrolyl group, indolyl group, pyrrolopyridyl group, furyl group, benzofuryl group, dihydrobenzofuryl group, dihydrobenzodioxyl group, thienyl group, benzothiophenyl group, oxazolyl group, benzoxazolyl group, isoxazolyl group, benzisoxazolyl group, thiazolyl group, benzothiazolyl group, isothiazolyl group, benzisothiazolyl group, pyrazolyl group, benzopyrazolyl group and imidazothiazolyl group. Particularly, a phenyl group, furyl group, indolyl group, benzofuryl group and benzothiophenyl group are preferable among them.

U, V, X and Y may be same or different from each other and, preferably, each independently represents a single bond, O, S, S(O), S(O₂), NRa, C(O), C(O)O, CRa=CRb or C=C (Ra and Rb each independently represents a hydrogen atom or a straight or branched alkyl group having 1 to 3 carbon atoms). A single bond is particularly preferable among them.

T is preferably N=C(Rd), wherein Rd is a hydrogen atom or a straight or branched alkyl group having 1 to 3 carbon atoms, and Rd is particularly preferably a hydrogen atom.

m is preferably 0.

n is preferably 0.

p is preferably 1.

q is preferably 1.

R₁ is preferably a hydrogen atom, a halogeno group, a hydroxyl group, an alkyl group which may have a substituent(s), an alkoxy group which may have a substituent(s), a carboxyl group, an acyl group which may have a substituent(s), an amino group which may have a substituent(s), an alkoxycarbonyl group which may have a substituent(s), an aliphatic cyclic group which may have a substituent(s), a benzyloxy group which may have a substituent(s), an aralkyl group which may have a substituent(s), an aryl group which may have a substituent(s) or a heterocyclic group which may have a substituent(s).

R₁ is also preferably a hydrogen atom, a halogeno group, a hydroxyl group, an alkyl group which may have a substituent(s), an alkoxy group which may have a substituent(s), a carboxyl group, an alkoxycarbonyl group which may have a substituent(s), an acyl group which may have a substituent(s), an amino group which may have a substituent(s), a benzyl group which may have a substituent(s), a benzyloxy group which may have a substituent(s), a phenyl group which may have a substituent(s), a pyridyl group which may have a substituent(s), a piperazino group which may have a substituent(s), a pyrrolidino group which may have a substituent(s), a morpholinyl group which may have a substituent(s), thiomorpholinyl group which may have a substituent(s) or an aminomorpholinyl group. A hydrogen atom, methyl group, phenyl group, pyridyl group and pyrrolidyl group are more preferable among them.

R₂ is preferably a hydrogen atom.

R₃ is preferably a hydrogen atom.

R₄ is preferably a hydrogen atom.

R₅ is preferably a hydrogen atom, a halogeno group, a hydroxyl group, a boronyl group which may have a substituent(s), an alkyl group which may have a substituent(s), a mercapto group, an alkoxy group which may have a substituent(s), an alkylthio group which may have a substituent(s), an alkylsulfonyl group which may have a substituent(s), an acyl group which may have a substituent(s), an acyloxy group which may have a substituent(s), an amino group which may have a substituent(s), a carboxyl group, an alkoxycarbonyl group which may have a substituent(s), a carbamoyl group which may have a substituent(s), a nitro group, a cyano group, a trifluoromethyl group, a sulfonic group, a sulfonamide group which may have a substituent(s), a sulfinamide group which may have a substituent(s), an alkenyl group which may have a substituent(s) or an alkynyl group which may have a substituent(s).

R₅ is also preferably a hydrogen atom, a halogeno group, a cyano group, a hydroxyl group, an amino group which may have a substituent(s), a boronyl group which may have a substituent(s), an alkyl group which may have a substituent(s), an alkenyl group which may have a substituent(s), an acyl group which may have a substituent(s) or an alkoxy group which may have a substituent(s). A hydrogen atom, methyl group, ethyl group, vinyl group, isopropyl group, isopropenyl group and acetyl group are more preferable among them.

Each of R₆, R₇, R₈ and R₉ is preferably a hydrogen atom.

The group of the following (II) having W represents a four- to six-membered cyclic amino group comprising a hetero atom represented by W, wherein W represents O, S or NRf (Rf represents a hydrogen atom or a straight or branched alkyl group having 1 to 3 carbon atoms): 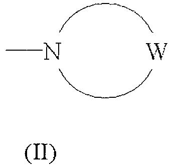 Examples thereof include a piperazino group, methylpiperazino group, morpholinyl group, thiazolidino group and thiomorpholinyl group, and a 4-morpholinyl group is preferable among them.

The group (II) having W may have a substituent(s) and, at that time, the group may have one to four substituent(s). The substituent(s) may be same or different from each other, and each one is independently selected from the group consisting of a halogeno group, hydroxyl group, alkyl group which may have a substituent(s), mercapto group, alkoxy group which may have a substituent(s), alkylthio group which may have a substituent(s), alkylsulfonyl group which may have a substituent(s), acyl group which may have a substituent(s), acyloxy group which may have a substituent(s), amino group which may have a substituent(s), carboxyl group, alkoxycarbonyl group which may have a substituent(s), carbamoyl group which may have a substituent(s), nitro group, cyano group, trifluoromethyl group, sulfonic group, aliphatic cyclic group which may have a substituent(s), alkenyl group which may have a substituent(s) and alkynyl group which may have a substituent(s). However, the group (II) having W is preferably a group without any substituent.

In the present invention, the imidazopyridazine compound of the formula (I) is preferably a compound constituted with the above mentioned preferable groups of each sign.

Further, in the present invention, the imidazopyridazine compound of the formula (I) or pharmaceutically acceptable salts thereof are preferably those as mentioned below:
The imidazopyridazine compound of the formula (I) or pharmaceutically acceptable salts thereof, wherein A is an aryl group or a heteroaryl group; E is an aryl group or a heteroaryl group; each of U, V, X and Y is a single bond; T is N=C(Rd); each of m and n is 0; each of p and q is 1;
R₁ is a hydrogen atom, a halogeno group, a hydroxyl group, a lower alkyl group, a mercapto group, an alkoxy group having 1 to 6 carbon atoms, an alkylthio group having 1 to 6 carbon atoms, a lower alkylsulfonyl group, an acyl group having 1 to 6 carbon atoms, an acyloxy group having 1 to 6 carbon atoms, an amino group, an alkylamino group having 1 to 6 carbon atoms, a carboxyl group, an alkoxycarbonyl group having 2 to 6 carbon atoms, a carbamoyl group, an alkylcarbamoyl group having 2 to 6 carbon atoms, a nitro group, a cyano group, a trifluoromethyl group, a sulfonic group, a sulfonamide group, a sulfinamide group, an aliphatic cyclic group, an alkenyl group having 2 to 6 carbon atoms, an alkynyl group having 2 to 6 carbon atoms, a benzyloxy group, an aryloxy group, an arylvinyl group, a heteroaryloxy group, an arylamino group, a heteroarylamino group, an arylethynyl group, an aralkyl group, an aryl group or a heterocyclic group; R₅ is a hydrogen atom, a halogeno group, a hydroxyl group, a boronyl group, a lower alkyl group, a mercapto group, an alkoxy group having 1 to 6 carbon atoms, an alkylthio group having 1 to 6 carbon atoms, a lower alkylsulfonyl group, an acyl group having 1 to 6 carbon atoms, an acyloxy group having 1 to 6 carbon atoms, an amino group, an alkylamino group having 1 to 6 carbon atoms, a carboxyl group, an alkoxycarbonyl group having 2 to 6 carbon atoms, a carbamoyl group, an alkylcarbamoyl group having 2 to 6 carbon atoms, a nitro group, a cyano group, a monofluoromethyl group, a difluoromethyl group, a trifluoromethyl group, a sulfonic group, a sulfonamide group, a sulfinamide group, an aliphatic cyclic group, an alkenyl group having 2 to 6 carbon atoms, an alkynyl group having 2 to 6 carbon atoms, a benzyloxy group, an aryloxy group, an arylvinyl group, a heteroaryloxy group, an arylamino group, a heteroarylamino group, an arylethynyl group, an aralkyl group, an aryl group or a heterocyclic group; and
the group (II) having W is a piperazino group, a methylpiperazino group, a morpholinyl group, a thiazolidino group or a thiomorpholinyl group.
The imidazopyridazine compound of the formula (I) or pharmaceutically acceptable salts thereof, wherein A is a phenyl group, a pyridyl group, a pyrazolyl group or a thienyl group; E is a phenyl group, a furyl group, an indolyl group, a pyrrolyl group, a benzofuryl group or a benzothiophenyl group; each of U, V, X and Y is a single bond; T is N=C(Rd) wherein Rd is a hydrogen atom; each of m and n is 0; each of p and q is 1;
R₁ is a hydrogen atom, a halogeno group, a hydroxyl group, a lower alkyl group, a mercapto group, an alkoxy group having 1 to 6 carbon atoms, an alkylthio group having 1 to 6 carbon atoms, a lower alkylsulfonyl group, an acyl group having 1 to 6 carbon atoms, an acyloxy group having 1 to 6 carbon atoms, an amino group, an alkylamino group having 1 to 6 carbon atoms, a carboxyl group, an alkoxycarbonyl group having 2 to 6 carbon atoms, a carbamoyl group, an alkylcarbamoyl group having 2 to 6 carbon atoms, a nitro group, a cyano group, a trifluoromethyl group, a sulfonic group, a sulfonamide group, a sulfinamide group, an aliphatic cyclic group having 1 to 6 carbon atoms, an alkenyl group having 2 to 6 carbon atoms, an alkynyl group having 2 to 6 carbon atoms, a phenyl group, a pyridyl group or a pyrrolidinyl group;
R₅ is a hydrogen atom, a halogeno group, a hydroxyl group, a boronyl group, a lower alkyl group, a mercapto group, an alkoxy group having 1 to 6 carbon atoms, an alkylthio group having 1 to 6 carbon atoms, a lower alkylsulfonyl group, an acyl group having 1 to 6 carbon atoms, an acyloxy group having 1 to 6 carbon atoms, an amino group, an alkylamino group having 1 to 6 carbon atoms, a carboxyl group, an alkoxycarbonyl group having 2 to 6 carbon atoms, a carbamoyl group, an alkylcarbamoyl group having 2 to 6 carbon atoms, a nitro group, a cyano group, a monofluoromethyl group, a difluoromethyl group, a trifluoromethyl group, a sulfonic group, a sulfonamide group, a sulfinamide group, an aliphatic cyclic group having 1 to 6 carbon atoms, an alkenyl group having 2 to 6 carbon atoms or an alkynyl group having 2 to 6 carbon atoms; and
the group (II) having W is a morpholinyl group.

In addition, in the present invention, the imidazopyridazine compound of the formula (I) or pharmaceutically acceptable salts thereof is preferably compounds mentioned in Examples. Particularly, compounds 3, 4, 8, 10, 20, 21, 22, 25, 26, 28, 29 and 30 as mentioned below are preferable among them.

As a representative example of the compound (I) of the present invention, a method for producing a compound (IA) is described below. The compound (IA) wherein T in the compound (I) of the present invention is N=C(Rd) can be synthesized by the following method, for example.

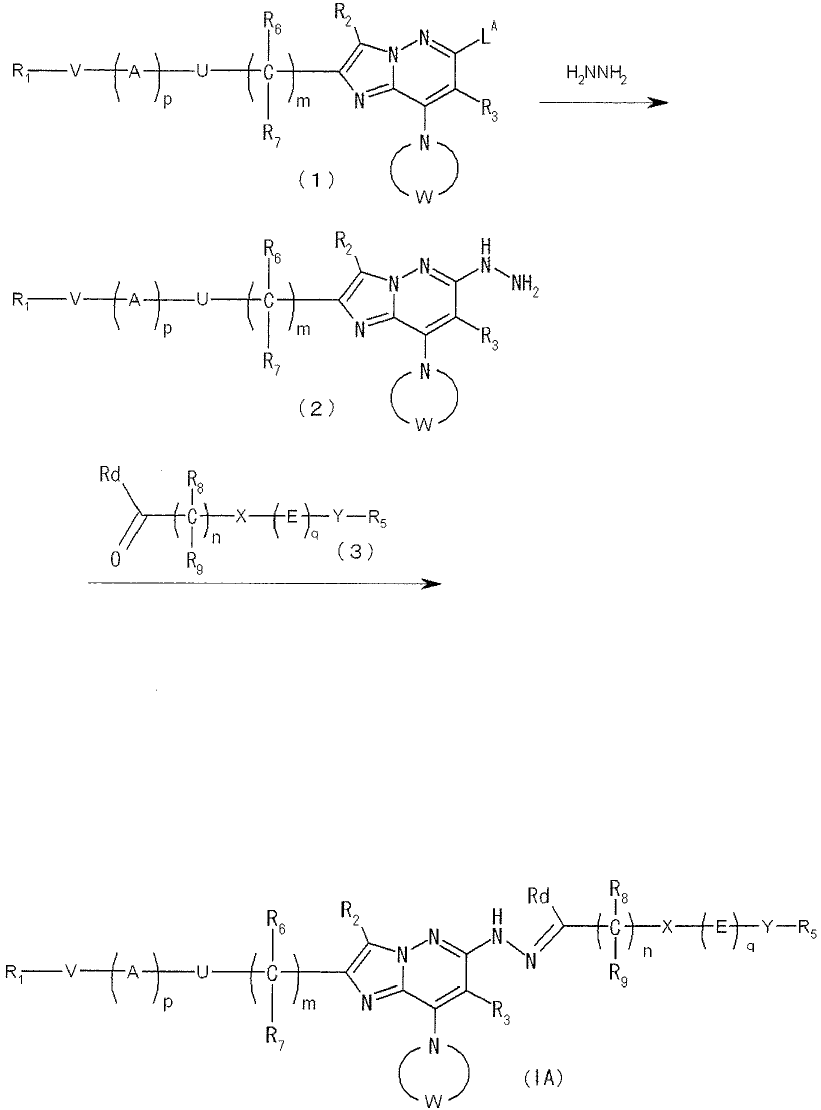

Hydrazine is reacted to the above compound (1) wherein L^{A} represents a leaving group to obtain a compound (2) wherein the leaving group L^{A} is substituted with a hydrazinyl group. Then, the substituted hydrazine (2) and aldehyde or ketone (3) are condensed to obtain a compound (IA).

Examples of the leaving group L^{A} include a chlorine atom. When L^{A} is a chlorine atom, the above substitution reaction with hydrazine is conducted to a mixture of the compound (1), lower alcohol such as ethanol or a polar solvent such as N,N-dimethylformamide and hydrazine monohydrate, by heating the mixture up to 50°C to 250°C. A water bath, a hot-water bath or a microwave is used for heating.

Further, the above condensation reaction of the substituted hydrazine with aldehyde or ketone can be conducted by stirring at room temperature the compound (2), the compound (3) and lower alcohol such as ethanol or a polar solvent such as N,N-dimethylformamide, and if necessary, by adding thereto an acid such as an acetic acid in a catalytic quantity.

The above compound (1) can be obtained by reacting an amine (5) to the following compound (4) wherein L^{A2} represents a leaving group, for example. 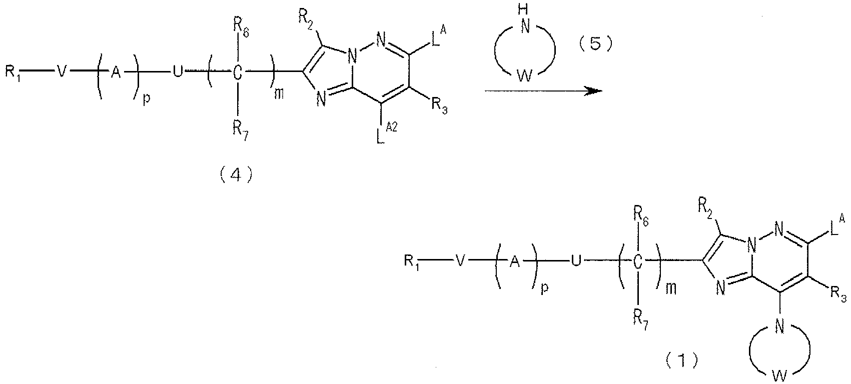 Here, L^{A} and L^{A2} may be same or different from each other. When L^{A} is a chlorine atom and L^{A2} is a bromine atom, the above substitution reaction with the amine can be conducted by stirring a mixture of the compound (4), the amine (5) and an ether solvent such as 1,4-dioxane at room temperature.

A compound (4-2) wherein, in the formula (4), L^{A} represents a chlorine atom and R₂ represents a hydrogen atom can be obtained by reacting the following compound (6) wherein L^{A3} represents a leaving group with a substituted pyridazine (7) in the presence of lower alcohol such as ethanol. Examples of the leaving group L^{A3} include a bromine atom.

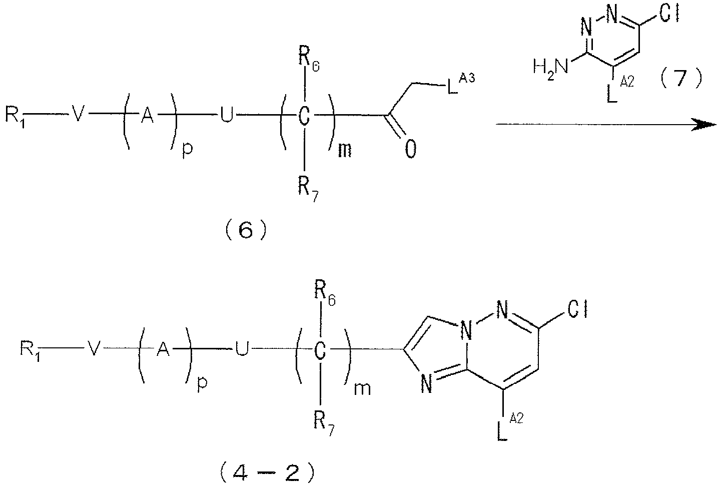

A compound (7-2) wherein L^{A2} in the above formula (7) is a bromine atom can be synthesized by reacting bromine to the following compound (8), for example.

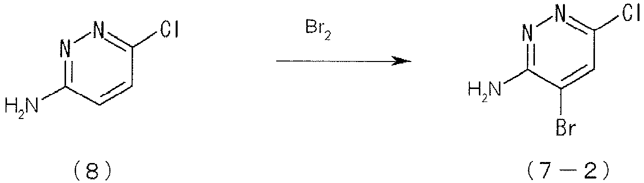

A compound (IB) wherein T in the compound (I) of the present invention is NRcC(RdRe) can be synthesized by reducing the compound (IA).

The reduction reaction of the compound (IA) can be conducted using a metal hydride such as lithium borohydride or using a catalyst such as palladium carbon in a reductive atmosphere such as in a hydrogen gas atmosphere.

In the above method for synthesizing each of the compounds (IA) and (IB), signs such as R₁ in a synthetic intermediate represent the same as those in the formula (I). However, when needed, a protected corresponding group(s) can be used instead of the group(s) in the formula (I), and the deprotection process can be added in an appropriate step. Further, a different group(s) can be used instead of the group(s) in the formula (I), and the adjustment can be conducted in an appropriate step such as addition of the process of changing a substituent(s).

In addition, when needed, the compound (I) of the present invention can be produced by conducting the adjustment, change and/or addition of the process easy for those skilled in the art to the above synthesizing methods or the synthesizing methods to be described in Examples.

The imidazopyridazine compound of the formula (I) of the present invention, the compound (IA), the compound (IB) or pharmaceutically acceptable salts thereof can be purified with the ordinary method such as silica gel chromatography, ion-exchange chromatography, reversed-phase high-performance liquid chromatography, affinity chromatography and recrystallization. The above chemical synthesis and subsequent purification are well known in this technical field.

The imidazopyridazine compound of the formula (I) of the present invention can be in the form of pharmaceutically acceptable salts thereof. In the case of the compound of the present invention which is sufficiently acidic, examples of the pharmaceutically acceptable salts thereof include ammonium salts thereof, alkali metal salts (such as sodium salts and potassium salts, as preferable examples), alkaline earth metal salts (such as calcium salts and magnesium salts, as preferable examples); and, as salts of an organic base, dicyclohexylamine salts, benzathine salts, N-methyl-D-glucan salts, hydramine salts, and salts of amino acids such as arginine and lysine. Further, in the case of the compound of the present invention which is sufficiently basic, examples of the pharmaceutically acceptable salts thereof include acid addition salts thereof, such as those of inorganic acids, e. g. a hydrochloric acid, sulfuric acid, nitric acid and phosphoric acid; or those of organic acids, e. g. an acetic acid, lactic acid, citric acid, tartaric acid, maleic acid, fumaric acid and monomethyl sulfate. In some cases, the salts can be wet salts or hydrates.

The present invention includes all isomers such as optical isomers and geometric isomers, hydrates, solvates or crystal forms.

The imidazopyridazine compound of the present invention or pharmaceutically acceptable salts thereof selectively inhibit IL-12/IL-23 production and do not significantly inhibit TNF- α production from activated macrophage. Namely, without significantly inhibiting TNF- α production, they significantly inhibit IL-12/IL-23. Therefore, they have a high selectivity as a therapeutic or preventive agent for diseases related to IL-12/IL-23 excessive production and does not bring concern about side effects associated with the compounds having an inhibiting activity against TNF- α production.

Further, in the preferable imidazopyridazine compound of the present invention or pharmaceutically acceptable salts thereof, the inhibiting activity thereof against IL-12/IL-23 production is not significantly reduced in whole blood, and thus, an extremely excellent medicinal effect can be provided in the administration to a human being in clinical practice.

The pharmaceutical composition or the therapeutic or preventive agent for diseases related to IL-12/IL-23 excessive production of the present invention can comprise, as the imidazopyridazine compound of the present invention or pharmaceutically acceptable salts thereof, any imidazopyridazine compound or pharmaceutically acceptable salts thereof included in the imidazopyridazine compound of the formula (I) or pharmaceutically acceptable salts thereof alone or in combinations with preferable two or more kinds thereof. Further, the composition and the agent can comprise any solid or liquid carrier or additives each of which is pharmaceutically, physiologically and experimentally acceptable.

Examples of the carrier include glucose, lactose, sucrose, starch, mannitol, dextrin, glycerides of fatty acids, polyethylene glycols, hydroxyethyl starch, ethylene glycols, polyoxyethylene sorbitan fatty acid ester, gelatin, albumin, amino acids, water and a normal saline solution. If necessary, common additives such as stabilizing agents, moisturizing agents, emulsifying agents, binders and tonicity agents can be preferably added to the pharmaceutical composition or the therapeutic or preventive agent for diseases related to IL-12/IL-23 excessive production of the present invention.

The above additives are not particularly limited as long as they are usually used for purposes corresponding to the purposes. Examples of the additives include flavoring agents, sugars, sweeteners, dietary fibers, vitamins, amino acids such as a monosodium glutamate (MSG), nucleic acids such as an inosine monophosphate (IMP), inorganic salts such as sodium chloride and water.

The pharmaceutical composition or the therapeutic or preventive agent for diseases related to IL-12/IL-23 excessive production of the present invention can be used in any form without limitation of properties, such as dry powder, paste and a solution.

The method of applying the pharmaceutical composition or the therapeutic or preventive agent for diseases related to IL-12/IL-23 excessive production of the present invention is not particularly limited, and any invasive or non-invasive administration such as oral administration and injection is applicable. Suppository or transdermal administration is also applicable. It is possible to administer an active ingredient by formulating it into a common pharmaceutical preparation form together with a solid or liquid pharmaceutical carrier that is suitable for the administration method such as oral administration and injection. Examples of the preparation form include solid agents such as tablets, granules, powders and capsules; liquid agents such as solutions, suspensions and emulsifying agents; and freeze-drying agents. These preparations can be prepared by common maneuver in pharmaceutical preparations. Further, any solid or liquid carrier or additives each of which is pharmaceutically and physiologically acceptable can be added to the pharmaceutical composition or the therapeutic or preventive agent for diseases related to IL-12/IL-23 excessive production of the present invention.

The usage amount of the pharmaceutical composition or the therapeutic or preventive agent for diseases related to IL-12/IL-23 excessive production of the present invention can be preferably adjusted corresponding to purposes. For example, in the case of orally administering the composition or the agent to a target, a total amount of the imidazopyridazine compound of the formula (I) or pharmaceutically acceptable salts thereof is preferably 0.0001mg to 5g per 1kg of body weight in one administration, more preferably 0.001mg to 1g, and further more preferably 0.01mg to 10mg. The frequency of administration is not particularly limited, and the composition or the agent can be administered once or several times per day.

The content of the imidazopyridazine compound of the formula (I) or pharmaceutically acceptable salts thereof in the pharmaceutical composition or the therapeutic or preventive agent for diseases related to IL-12/IL-23 excessive production of the present invention is not particularly limited only if it fits the usage amount mentioned above. The content thereof is preferably 0.000001 to 99.9999 weight% per dry weight, more preferably 0.00001 to 99.999 weight%, and particularly preferably 0.0001 to 99.99 weight%.

The pharmaceutical composition or the therapeutic or preventive agent for diseases related to IL-12/IL-23 excessive production of the present invention can further contain one or more kinds of a known substance(s) that can exert clinically desired effects.

The pharmaceutical composition or the therapeutic or preventive agent for diseases related to IL-12/IL-23 excessive production of the present invention can be used against any disease or state including the diseases related to IL-12/IL-23 excessive production against which the composition or the agent can exert clinically desired therapeutic or preventive effects. Examples of the diseases related to IL-12/IL-23 excessive production include multiple sclerosis, systemic sclerosis, sepsis, myasthenia gravis, autoimmune neurological disease, Guillain-Barre syndrome, autoimmune uveitides, autoimmune hemolytic anemia, pernicious anemia, autoimmune thrombocytopenia, temporal arteritis, antiphospholipid syndrome, vasculitis, Wegener's granulomatosis, Behcet's disease, psoriasis, psoriatic arthritis, herpetic dermatitis, pemphigus vulgaris, vitiligo, Crohn's disease, ulcerative colitis, interstitial fibroid lung, myelofibrosis, hepatic fibrosis, myocarditis, autoimmune thyroid disease (Graves' disease, Hashimoto's disease), primary biliary cirrhosis, autoimmune hepatitis, immune-mediated diabetes mellitus, autoimmune oophoritis and orchitis, autoimmune adrenalitis, rheumatoid arthritis, juvenile rheumatoid arthritis, systemic lupus erythematosus, scleroderma, polymyositis, dermatomyositis, spondyloarthropathy, ankylosing spondylitis, Sjögren's syndrome, graft versus host disease and ischemic vascular disorder, though they are not limited to these diseases.

The composition or the agent of the present invention can be preferably used against Crohn's disease, ulcerative colitis, rheumatoid arthritis, multiple sclerosis, psoriasis, psoriatic arthritis, sepsis, primary biliary cirrhosis, and autoimmune uveitides.

Further, the pharmaceutical composition of the present invention can be used as a preventive or therapeutic agent for inflammatory diseases including the above diseases related to IL-12/IL-23 excessive production.

### Examples

Next, Examples will further illustrate the present invention. They only explain the present invention and do not particularly limit the invention.

In the specification, the ordinary method indicates the method generally used as a chemical operation such as liquid separation, drying, filtration and concentration.

The following examples will further illustrate synthesis of the representative compounds of the present invention. They only explain the compounds of the present invention and do not particularly limit them.

Meanwhile, in a referential example, examples and test examples, room temperature indicates 1 o 30°C, and % indicates weight%, if not otherwise specified.

In the following referential example and examples, the measurement of 1H-NMR and the reaction with a microwave were conducted using the following equipments:
1H-NMR: DPX300 (300MHz), Bruker
Microwave: Initiator 60EXP, Biotage

### Example 1: Synthesis of N-(3-methyl-benzylidene)-N'-(8-mor-pholin-4-yl-2-phenyl-imidazo[1,2-b]pyridazin-6-yl)-hydrazine (Compound 1)

### (Step 1)

### 6-Chloro-8-morpholin-4-yl-2-phenyl-imidazo[1,2-b]pyridazine

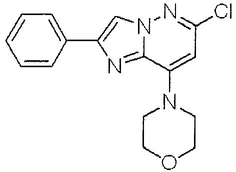

4-Bromo-6-chloropyridazin-3-ylamine (1.11g, 5.33mmol) was dissolved in ethanol (10mL). 2-Bromoacetophenone (1.17g, 5.86mmol) was added thereto and stirred at 70°C overnight, and then the solvent was removed from the reaction liquid. 1,4-Dioxane (10mL) and morpholine (1.0mL, 12mmol) were added to the obtained residue and stirred at room temperature for 90 minutes. Then, the solvent was removed from the reaction mixture, and the residue was diluted with a saturated sodium hydrogen carbonate aqueous solution and extracted with an ethyl acetate. The extract together with the extraction liquid was dried with anhydrous sodium sulfate, and then the solvent was removed therefrom. The obtained residue was washed with methanol to obtain a title compound (602mg, 36%). The characteristic value of the compound is shown below.
¹H-NMR (300 MHz, CDCl₃): δ 3.92-3.95 (m, 4H), 4.10-4.13 (m, 4H), 6.08 (s, 1H), 7.33 (m, 1H), 7.41-7.46 (m, 2H), 7.88-7.92 (m, 2H), 8.02 (s, 1H); MS (ESI) m/z 315 (M+H)⁺.

### (Step 2)

### N-(3-methyl-benzylidene)-N'-(8-morpholin-4-yl-2-phenyl-imidazo[1,2-b]pyrid azin-6-yl)-hydrazine (Compound 1)

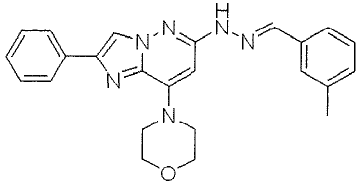

6-Chloro-8-morpholin-4-yl-2-phenyl-imidazo[1,2-b]pyridazine (30.0mg, 0.0953mmol) was dissolved in dimethylformamide (2mL). Potassium carbonate (132mg, 0.953mmol) and hydrazine monohydrate (46.2µL, 0.953mmol) were added thereto and stirred at 160°C for 80 minutes under irradiating microwave. Thus obtained reaction liquid was diluted with water and extracted with an ethyl acetate. The extract together with the extraction liquid was dried with anhydrous sodium sulfate, and then the solvent was removed therefrom. The obtained residue was purified with reversed-phase HPLC and dechlorinated to obtain (8-morpholin-4-yl-2-phenyl-imidazo[1,2-b]pyridazin-6-yl)-hydrazine (15.0mg, 51%). The compound was dissolved in ethanol (2mL), and 3-methylbenzaldehyde (5.7µL, 0.0483mmol) was added thereto and stirred at room temperature for 1 hour. Then, the reaction liquid was filtered, and the obtained solid material was washed with methanol to obtain a title compound (5.0mg, 25%). The characteristic value of the compound is shown below.
¹H-NMR (300 MHz, DMSO): δ 2.33 (s, 3H), 3.82-3.85 (m, 4H), 3.96-3.99 (m, 4H), 6.14 (s, 1H), 7.14 (m, 1H), 7.24-7.31 (m, 2H), 7.37-7.50 (m, 4H), 7.92 (d, 2H, J=7.0 Hz), 7.97 (s, 1H), 8.32 (s, 1H), 10.88 (s, 1H); MS (ESI) m/z 413 (M+H)+.

### Example 2: Synthesis of N-(1H-indol-3-yl-methylidene)-N'-(8-morpholin-4-yl-2-phenyl-imidazo[1,2-b]pyridazin-6-yl)-hydrazine compound 2)

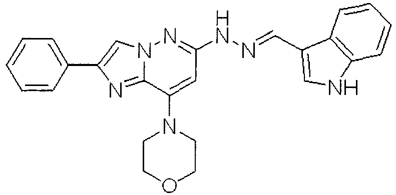

(8-Morpholin-4-yl-2-phenyl-imidazo[1,2-b]pyridazin-6-yl)-hydrazine (20.1mg, 0.0648mmol) was dissolved in ethanol. 1H-indole-3-carboxyaldehyde (10.0mg, 0.0648mmol) was added thereto and stirred at room temperature for 3 hours. Then, the reaction liquid was filtered, and the obtained solid material was washed with methanol to obtain a title compound (16.1mg, 92%). The characteristic value of the compound is shown below.
¹H-NMR (300 MHz, DMSO): δ 3.87-3.89 (m, 4H), 3.96-3.98 (m, 4H), 6.51 (s, 1H), 7.13-7.20 (m, 2H), 7.26 (m, 1H), 7.37-7.42 (m, 3H), 7.68 (d, 1H, J=2.6 Hz), 7.93 (d, 2H, J=7.9 Hz), 8.21 (m, 1H), 8.22 (s, 1H), 8.29 (s, 1H), 10.52 (s, 1H), 11.39 (s, 1H); MS (ESI) m/z 438 (M+H)⁺.

### Example 3: Synthesis of N-(3-methyl-benzylidene)-N'-(8-morpholin-4-yl-2-pyridin-3-yl-imidazo[1,2-b]pyridazin-6-yl)-hydrazine (Compound 3)

### (Step 1)

### 6-Chloro-8-morpholin-4-yl-2-pyridin-3-yl-imidazo[1,2-b]pyridazine

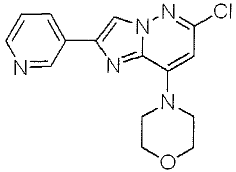

4-Bromo-6-chloropyridazin-3-ylamine (500mg, 2.40mmol) was dissolved in ethanol (10mL). 2-Bromo-1-pyridin-3-yl-ethanone hydrobromate (809mg, 2.88mmol) was added thereto and stirred with heating under reflux overnight, and then the solvent was removed from the reaction liquid. 1,4-Dioxane (10mL) and morpholine (419 µL, 4.80mmol) were added to the obtained residue and stirred at room temperature for 3 hours. Then, the solvent was removed from the reaction mixture, and the residue was washed with diethyl ether to obtain a title compound (252mg, 33%). The characteristic value of the compound is shown below.
¹H-NMR (300 MHz, DMSO): δ 3.76-3.80 (m, 4H), 4.10-4.14 (m, 4H), 6.45 (s, 1H), 7.46 (ddd, 1H, J=0.6, 4.7, 7.9 Hz), 8.29 (ddd, 1H, J=1.8, 2.1, 7.9 Hz), 8.51 (dd, 1H, J=1.8, 4.7 Hz), 9.16 (dd, 1H, J=0.6, 2.1 Hz); MS (ESI) m/z 316 (M+H)⁺.

### (Step 2)

### N-(3-methyl-benzylidene)-N'-(8-morpholin-4-yl-2-pyridin-3-yl-imidazo[1,2-b] pyridazin-6-yl)-hydrazine (Compound 3)

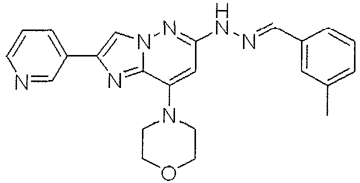

6-Chloro-8-morpholin-4-yl-2-pyridin-3-yl-imidazo[1,2-b]pyridazine (108mg, 0.342mmol) was dissolved in dimethylformamide (2mL). Potassium carbonate (94.5mg, 0.684mmol) and hydrazine monohydrate (166 µL, .3.42mmol) were added thereto and stirred at 170°C for 80 minutes under irradiating microwave. Thus obtained reaction liquid was diluted with water and extracted with an ethyl acetate. The extract together with the extraction liquid was dried with anhydrous sodium sulfate, and then the solvent was removed therefrom. The obtained residue was dissolved in ethanol (2mL), and 3-methylbenzaldehyde (48.0 µL, 0.376mmol) was added thereto and stirred at room temperature for 2 hours. Then, the solvent was removed from the reaction liquid, and the obtained residue was purified with reversed-phase HPLC and dechlorinated to obtain a title compound (9.8mg, 7.0%). The characteristic value of the compound is shown below.
¹H-NMR (300 MHz, DMSO): δ 2.33 (s, 3H), 3.82-3.85 (m, 4H), 3.95-3.98 (m, 4H), 6.43 (s, 1H), 7.15 (m, 1H), 7.29 (t, 1H, J=7.6 Hz), 7.40-7.45 (m, 2H), 7.49 (m, 1H), 7.98 (s, 1H), 8.25 (dt, 1H, J=7.9, 1.9 Hz), 8.46 (s, 1H), 8.46 (m, 1H), 9.13 (d, 1H, J=2.3 Hz), 10.91 (s, 1H); MS (ESI) m/z 414 (M+H)⁺.

### Example 4: Synthesis of N-(3-methyl-benzylidene)-N'-(8-morpholin-4-yl-2-pyridin-4-yl-imidazo[1,2-b]pyridazin-6-yl)-hydrazine (Compound 4)

### (Step 1)

### 6-Chloro-8-morpholin-4-yl-2-pyridin-4-yl-imidazo[1,2-b]pyridazine

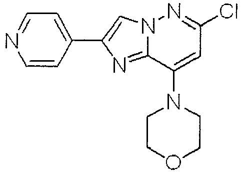

4-Bromo-6-chloropyridazin-3-ylamine (500mg, 2.40mmol) was dissolved in ethanol (15mL), and 2-Bromo-1-pyridin-4-yl-ethanone hydrobromate (1.01g, 3.60mmol) was added thereto and stirred with heating under reflux overnight. About a half of the solvent was removed from the reaction liquid, and an ethyl acetate was added thereto and then filtered. The obtained solid material was washed with diethyl ether. Then, the solid material was dissolved in 1,4-dioxane (10mL), and morpholine (419 µL, 4.80mmol) was added thereto and stirred at room temperature for 2 hours. The solvent was removed from the reaction mixture, and the residue was diluted with water and extracted with an ethyl acetate. The extract together with the extraction liquid was dried with anhydrous sodium sulfate, and then the solvent was removed therefrom. The obtained solid material was washed with diethyl ether to obtain a title compound (337mg, 44%). The characteristic value of the compound is shown below.
¹H-NMR (300 MHz, DMSO): δ 3.77-3.80 (m, 4H), 4.11-4.14 (m, 4H), 6.46 (s, 1H), 7.90 (d, 2H, J=6.2 Hz), 8.61 (d, 2H, J=6.2 Hz), 8.82 (s, 1H); MS (ESI) m/z 316 (M+H)⁺.

### (Step 2)

### (8-Morpholin-4-yl-2-pyridin-4-yl-imidazo[1,2-b]pyridazin-6-yl)-hydrazine

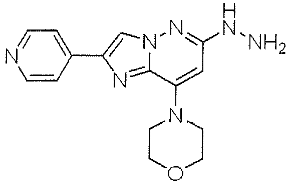

6-Chloro-8-morpholin-4-yl-2-pyridin-4-yl-imidazo[1,2-b]pyridazine (200 mg, 0.633 mol) was dissolved in N-methylpyrrolidone (5mL). Potassium carbonate (101mg, 1.27mmol) and hydrazine monohydrate (307 µL, 6.33mmol) were added thereto and stirred in a sealed tube at 150°C for 3 hours. Thus obtained reaction liquid was diluted with water and extracted with an ethyl acetate. The extract together with the extraction liquid was dried with anhydrous sodium sulfate, and then the solvent was removed therefrom. The obtained solid material was washed with diethyl ether to obtain a title compound (80.9mg, 41%). The characteristic value of the compound is shown below.
¹H-NMR (300 MHz, DMSO): δ 3.79 (br, 8H), 4.08 (br, 2H), 5.87 (s, 1H), 7.55 (br, 1H), 7.83 (d, 2H, J=6.2 Hz), 8.48 (s, 1H), 8.54 (d, 2H, J=6.2 Hz); MS (ESI) m/z 312 (M+H)⁺.

### (Step 3)

### N-(3-methyl-benzylidene)-N'-(8-morpholin-4-yl-2-pyridin-4-yl-imidazo[1,2-b] pyridazin-6-yl)-hydrazine (Compound 4)

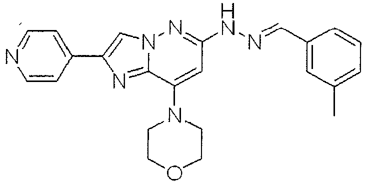

(8-Morpholin-4-yl-2-pyridin-4-yl-imidazo[1,2-b]pyridazin-6-yl)-hydrazine (40.0mg, 0.129mmol) was dissolved in ethanol (2mL). 3-Methylbenzaldehyde (15.2 µL, 0.129mmol) was added thereto and stirred at room temperature for 2 hours. Then, the reaction liquid was filtered, and the obtained solid material was washed with diethyl ether to obtain a title compound (39.5mg, 74%). The characteristic value of the compound is shown below.
¹H-NMR (300 MHz, DMSO): δ 2.33 (s, 3H), 3.83-3.85 (m, 4H), 3.95-3.97 (m, 4H), 6.44 (s, 1H), 7.15 (d, 1H, J=7.6 Hz), 7.29 (t, 1H, J=7.6 Hz), 7.44 (s, 1H), 7.49 (d, 1H, J=7.6 Hz), 7.89 (d, 2H, J=6.3 Hz), 7.99 (s, 1H), 8.58 (d, 2H, J=6.3 Hz), 8.59 (s, 1H), 10.94 (s, 1H); MS (ESI) m/z 414 (M+H)⁺.

### Example 5: Synthesis of N-(3-methyl-benzylidene)-N'-[2-(5-methyl-1-phenyl-1H-pyrazol-4-yl)-8-morpholin-4-yl-imidazo[1,2-b]pyridazin-6-yl]-hydrazine (Compound 5)

### (Step 1)

### 6-Chloro-2-(5-methyl-1-phenyl-1H-pyrazol-4-yl)-8-morpholin-4-yl -imidazo[1,2-b] pyridazine

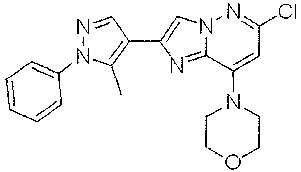

4-Bromo-6-chloropyridazin-3-ylamine (500mg, 2.40mmol) was dissolved in ethanol (10mL). 2-Bromo-1-(5-methyl-1-phenyl-1H-pyrazol-4-yl)-1-ethanone (803mg, 2.88mmol) was added thereto and stirred with heating under reflux overnight, and then the solvent was removed from the reaction liquid. The obtained residue was diluted with a saturated sodium hydrogen carbonate aqueous solution and extracted with an ethyl acetate. The extract together with the extraction liquid was dried with anhydrous sodium sulfate, and then the solvent was removed therefrom. The obtained residue was purified with NH- silica gel column chromatography (methylene chloride). The obtained solid material was dissolved in 1,4-dioxane (10mL), and morpholine (419 µL, 4.80mmol) was added thereto and stirred at room temperature for 3 hours. The solvent was removed from the reaction mixture, and the residue was diluted with water and extracted with an ethyl acetate. The extract together with the extraction liquid was dried with anhydrous sodium sulfate, and then the solvent was removed therefrom. The obtained solid material was washed with diethyl ether to obtain a title compound (376mg, 40%). The characteristic value of the compound is shown below.
¹H-NMR (300 MHz, CDCl₃): δ 2.57 (s, 3H), 3.91-3.94 (m, 4H), 4.07-4.10 (m, 4H), 6.09 (s, 1H), 7.37-7.51 (m, 5H), 7.84 (s, 1H), 7.98 (s, 1H); MS (ESI) m/z 395 (M+H)⁺.

### (Step 2)

### N-(3-methyl-benzylidene)-N'-[2-(5-methyl-1-phenyl-1H-pyrazol-4-yl)-8-morp holin-4-yl- imidazo[1,2-b]pyridazin-6-yl]-hydrazine (Compound 5)

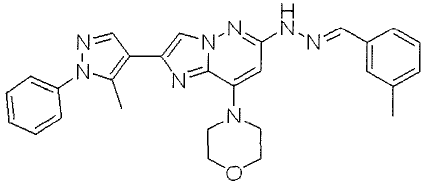

6-Chloro-2-(5-methyl-1-phenyl-1H-pyrazol-4-yl)-8-morpholin-4-yl-imidazo[1, 2-b] pyridazine (198mg, 0.501mmol) was dissolved in N-methylpyrrolidone (3mL). Potassium carbonate (76.2mg, 0.551mmol) and hydrazine monohydrate (243 µL, 5.01mmol) were added thereto and stirred at 200°C for 50 minutes under irradiating microwave. Thus obtained reaction liquid was diluted with water and extracted with an ethyl acetate. The extract together with the extraction liquid was dried with anhydrous sodium sulfate, and then the solvent was removed therefrom. The obtained residue was dissolved in ethanol (3mL), and 3-methylbenzaldehyde (117 µL, 1.50mmol) was added thereto and stirred at room temperature overnight. Then, the solvent was removed from the reaction liquid, and the obtained residue was purified with reversed-phase HPLC and dechlorinated to obtain a title compound (30.3mg, 12%). The characteristic value of the compound is shown below.
¹H-NMR (300 MHz, DMSO): δ 2.34 (s, 3H), 2.58 (s, 3H), 3.81-3.84 (m, 4H), 3.94-3.96 (m, 4H), 6.41 (s, 1H), 7.15 (d, 1H, J=7.6 Hz), 7.29 (t, 1H, J=7.6 Hz), 7.44-7.55 (m, 7H), 7.98 (s, 1H), 8.00 (s, 1H), 8.03 (s, 1H), 10.85 (s, 1H); MS (ESI) m/z 493 (M+H)⁺.

### Example 6: Synthesis of N-(1H-indol-3-yl-methylidene)-N'-(8-morpholin-4-yl-2-pyridin-4-yl-imidazo[1,2-b]pyridazin-6-yl)-hydrazine (Compound 6)

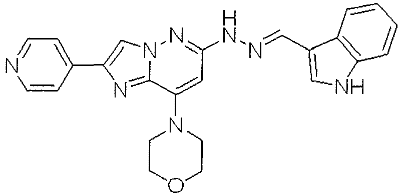

6-Chloro-8-morpholin-4-yl-2-pyridin-4-yl-imidazo[1,2-b]pyridazine (115mg, 0.364mol) was dissolved in N-methylpyrrolidone (3mL). Potassium carbonate (122 µL, 2.51mmol) and hydrazine monohydrate (307 µL, 6.33mmol) were added thereto and stirred in a sealed tube at 150°C for 1 hour. Thus obtained reaction liquid was diluted with water and extracted with an ethyl acetate. The extract together with the extraction liquid was dried with anhydrous sodium sulfate, and then the solvent was removed therefrom. The obtained residue was purified with reversed-phase HPLC. The obtained solid material was dissolved in ethanol (3mL), and 1H-indole-3-carboxyaldehyde (52.8mg, 0.364mmol) was added thereto and stirred at room temperature for 2 hours. A saturated sodium hydrogen carbonate aqueous solution was added to the reaction liquid and filtered. Then, the obtained solid material was washed with methanol to obtain a title compound (100mg, 63%). The characteristic value of the compound is shown below.
¹H-NMR (300 MHz, DMSO): δ 3.86-3.89 (m, 4H), 3.96-3.99 (m, 4H), 6.53 (s, 1H), 7.13 -7.21 (m, 2H), 7.42 (m, 1H), 7.69 (d, 1H, J=2.6 Hz), 7.88 (d, 2H, J=6.0 Hz), 8.21 (m, 1H), 8.24 (s, 1H), 8.54 (s, 1H), 8.56 (d, 2H, J=6.0 Hz), 10.60 (s, 1H), 11.41 (s, 1H); MS (ESI) m/z 439 (M+H)⁺.

### Example 7: Synthesis of N-(3-methyl-benzylidene)-N'-[8-morpholin-4-yl-2-(4-pyrrolidin-1-yl-phenyl)-imidazo[1,2-b]pyridazin-6-yl-hydrazine (Compound 7)

### (Step 1)

### 6-Chloro-8-morpholin-4-yl-2-(4-pyrrolidin-1-yl-phenyl)-imidazo[1,2-b]pyridaz ine

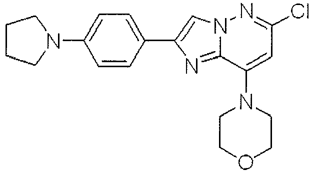

4-Bromo-6-chloropyridazin-3-ylamine (500mg, 2.40mmol) was dissolved in ethanol (10mL). 2-Bromo-1-(4-pyrrolidin-1-yl-phenyl)-ethanone (772mg, 2.88mmol) was added thereto and stirred with heating under reflux overnight. The solvent was removed from the reaction liquid, and the obtained solid material was washed with an ethyl acetate. Then, the solid material was dissolved in 1,4-dioxane (10mL), and morpholine (419 µL, 4.80mmol) was added thereto and stirred at room temperature for 2 hours. The solvent was removed from the reaction mixture, and the residue was diluted with water and extracted with an ethyl acetate. The extract together with the extraction liquid was dried with anhydrous sodium sulfate, and then the solvent was removed therefrom. The obtained solid material was washed with diethyl ether to obtain a title compound (487mg, 53%). The characteristic value of the compound is shown below.
¹H-NMR (300 MHz, CDCl₃): δ 2.00-2.05 (m, 4H), 3.31-3.36 (m, 4H), 3.91-3.94 (m, 4H), 4.07-4.10 (m, 4H), 6.04 (s, 1H), 6.61 (d, 2H, J=8.8 Hz), 7.76 (d, 2H, J=8.8 Hz), 7.88 (s, 1H); MS (ESI) m/z 384 (M+H)⁺.

### (Step 2)

### N-(3-methyl-benzylidene)-N'-[8-morpholin-4-yl-2-(4-pyrrolidin-1-yl-phenyl)-i midazo[1,2-b]pyridazin-6-yl]-hydrazine (Compound 7)

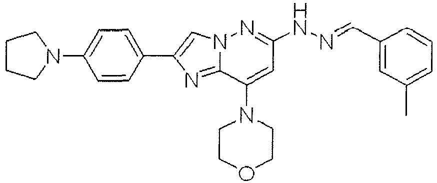

6-Chloro-8-morpholin-4-yl-2-(4-pyrrolidin-1-yl-phenyl)-imidazo[1,2-b]pyridaz ine (189mg, 0.492mmol) was dissolved in N-methylpyrrolidone (3mL). Potassium carbonate (74.8mg, 0.541mmol) and hydrazine monohydrate (239 µL, 4.92mmol) were added thereto and stirred at 200°C for 30 minutes under irradiating microwave. Thus obtained reaction liquid was diluted with water and extracted with an ethyl acetate. The extract together with the extraction liquid was dried with anhydrous sodium sulfate, and then the solvent was removed therefrom. The obtained residue was dissolved in ethanol (3mL), and 3-methylbenzaldehyde (58.0 µL, 0.492mmol) was added thereto and stirred at room temperature overnight. Then, the reaction liquid was filtered, and the obtained solid material was washed with ethanol to obtain a title compound (90.9mg, 39%). The characteristic value of the compound is shown below.
¹H-NMR (300 MHz, DMSO): δ 2.33 (s, 3H), 3.23-3.30 (m, 8H), 3.82-3.85 (m, 4H), 3.95-3.96 (m, 4H), 6.38 (s, 1H), 6.56 (d, 2H, J=8.8 Hz), 7.14 (d, 1H, J=7.6 Hz), 7.29 (t, 1H, J=7.6 Hz), 7.43 (s, 1H), 7.48 (d, 1H, J=7.6 Hz), 7.72 (d, 2H, J=8.8 Hz), 7.96 (s, 1H), 8.07 (s, 1H), 10.81 (s, 1H); MS (ESI) m/z 482 (M+H)⁺.

### Example 8: Synthesis of N-(3-methyl-benzylidene)-N'-[8-morpholin-4-yl-2-(4-pyrrolidin-1-yl-phenyl)-imidazo[1,2-b]pyridazin-6-yl]-hydrazine (Compound 8)

### (Step 1)

### 6-Chloro-8-morpholin-4-yl-2-(5-pyridin-2-yl-thiophen-2-yl)-imidazo[1,2-b]pyr idazine

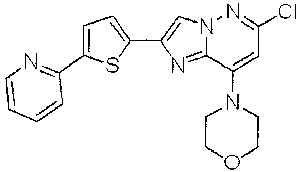

4-Bromo-6-chloropyridazin-3-ylamine (501mg, 2.40mmol) was dissolved in ethanol (15mL). 2-Bromo-1-(5-pyridin-2-yl-thiophen-2-yl)-ethanone (767mg, 2.88mmol) was added thereto and stirred with heating under reflux overnight. The solvent was removed from the reaction liquid, and the obtained solid material was washed with an ethyl acetate. Then, the solid material was dissolved in 1,4-dioxane (10mL), and morpholine (419 µL, 4.80mmol) was added thereto and stirred at room temperature overnight. The solvent was removed from the reaction mixture, and the residue was diluted with water and extracted with an ethyl acetate. The extract together with the extraction liquid was dried with anhydrous sodium sulfate, and then the solvent was removed therefrom. The obtained solid material was washed with diethyl ether to obtain a title compound (487mg, 53%). The characteristic value of the compound is shown below.
¹H-NMR (300 MHz, CDCl₃): δ 3.91-3.94 (m, 4H), 4.07-4.10 (m, 4H), 6.07 (s, 1H), 7.15 (m, 1H), 7.41 (d, 1H, J=3.8 Hz), 7.54 (d, 1H, J=3.8 Hz), 7.68-7.70 (m, 2H), 7.95 (s, 1H), 8.58 (m, 1H), MS (ESI) m/z 398 (M+H)⁺.

### (Step 2)

### N-(3-methyl-benzylidene)-N'-[8-morpholin-4-yl-2-(5-pyridin-2-yl-thiophen-2-yl)-imidazo[1,2-b]pyridazin-6-yl]-hydrazine (Compound 8)

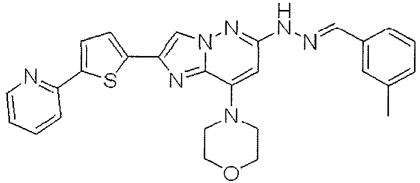

6-Chloro-8-morpholin-4-yl-2-(5-pyridin-2-yl-thiophen-2-yl)-imidazo[1,2-b] pyridazine (100mg, 0.252mol) was dissolved in N-methylpyrrolidone (3mL). Potassium carbonate (69.4mg, 0.502mmol) and hydrazine monohydrate (122 µL, 2.51mmol) were added thereto and stirred in a sealed tube at 150°C for 2 hours. Thus obtained reaction liquid was diluted with water and extracted with an ethyl acetate. The extract together with the extraction liquid was dried with anhydrous sodium sulfate, and then the solvent was removed therefrom. The obtained residue was dissolved in ethanol (3mL), and 3-methylbenzaldehyde (29.6 µL 0.251mmol) was added thereto and stirred at room temperature overnight. Then, the reaction liquid was filtered, and the obtained solid material was washed with ethanol to obtain a title compound (65.6mg, 53%). The characteristic value of the compound is shown below.
¹H-NMR (300 MHz, DMSO): δ 2.34 (s, 3H), 3.84-3.86 (m, 4H), 3.94-3.97 (m, 4H), 6.43 (s, 1H), 7.15 (d, 1H, J=7.3 Hz), 7.23-7.32 (m, 2H), 7.44 (s, 1H), 7.49-7.50 (m, 2H), 7.76 (d, 1H, J=3.8 Hz), 7.81 (dt, 1H, J=1.5, 7.6 Hz), 7.91 (d, 1H, J=8.2 Hz), 7.98 (s, 1H), 8.50 (m, 1H), 10.92 (s, 1H); MS (ESI) m/z 496 (M+H)⁺.

### Example 9: Syntheses of N-benzofuran-3-yl-methylidene-N'-(8-morpholin-4-yl-2-pyridin-4-yl-imidazo[1.2-b]pyridazin-6-yl)-hydrazine (Compound 9)

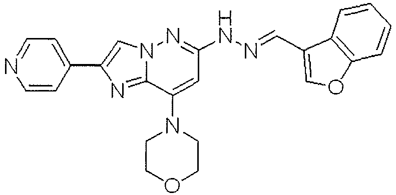

6-Chloro-8-morpholin-4-yl-2-pyridin-4-yl-imidazo[1,2-b] pyridazine (65.6mg, 0.208mol) was dissolved in N-methylpyrrolidone (2mL). Potassium carbonate (57.5mg, 0.416mmol) and hydrazine monohydrate (101 µL, 2.08mmol) were added thereto and stirred in a sealed tube at 150°C for 2 hours. Thus obtained reaction liquid was diluted with water and extracted with an ethyl acetate. The extract together with the extraction liquid was dried with anhydrous sodium sulfate, and then the solvent was removed therefrom. The obtained residue was dissolved in ethanol (3mL), and benzofuran-3-carboxyaldehyde (30.4mg, 0.208mmol) was added thereto and stirred at room temperature for 3 hours. Then, the reaction liquid was filtered, and the obtained solid material was washed with diethyl ether to obtain a title compound (37.2mg, 41%). The characteristic value of the compound is shown below.
¹H-NMR (300 MHz, DMSO): δ 3.87-3.89 (m, 4H), 3.98-4.00 (m, 4H), 6.50 (s, 1H), 7.40-7.43 (m, 2H), 7.64 (m, 1H), 7.88 (d, 2H, J=6.2 Hz), 8.21-8.24 (m, 2H), 8.37 (s, 1H), 8.57 (d, 2H, J=6.2 Hz), 8.59 (s, 1H), 11.03 (s, 1H); MS (ESI) m/z 440 (M+H)⁺.

### Example 10: Synthesis of N-(3-ethyl-benzylidene)-N'-(8-morpholin-4-yl-2-pyridin-4-yl-imidazo[1,2-b]pyridazin-6-yl)-hydrazine (Compound 10)

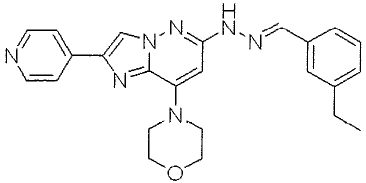

(8-Morpholin-4-yl-2-pyridin-4-yl-imidazo[1,2-b]pyridazin-6-yl)-hydrazine (21.6mg, 0.0694mmol) was dissolved in ethanol (2mL). 3-Ethylbenzaldehyde (10.2mg, 0.0763mmol) was added thereto and stirred at room temperature for 2 hours. Then, the reaction liquid was filtered, and the obtained solid material was washed with diethyl ether to obtain a title compound (24.4mg, 82%). The characteristic value of the compound is shown below.
¹H-NMR (300 MHz, DMSO): δ 1.20 (t, 3H, J=7.6 Hz), 2.62 (q, 2H, J=7.6 Hz), 3.83-3.85 (m, 4H), 3.95-3.97 (m, 4H), 6.44 (s, 1H), 7.18 (d, 1H, J=7.6 Hz), 7.32 (t, 1H, J=7.6 Hz), 7.46 (s, 1H), 7.52 (d, 1H, J=7.6 Hz), 7.87 (d, 2H, J=5.9 Hz), 8.00 (s, 1H), 8.56-8.57 (m, 3H), 10.95 (s, 1H); MS (ESI) m/z 428 (M+H)⁺.

### Example 11: Synthesis of N-benzo[b]thio-ohen-3-yl-methylidene-N'-(8-morpholin-4-yl-2-pyridin-4-yl-imidazo[1,2-b]pyridazin-6-yl)-hydrazine (Compound 11)

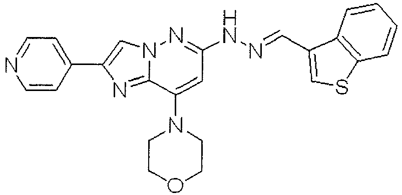

(8-Morpholin-4-yl-2-pyridin-4-yl-imidazo[1,2-b]pyridazin-6-yl)-hydrazine (16.2mg, 0.0520mmol) was dissolved in ethanol (2mL). Thionaphthene-3-carboxyaldehyde (9.3mg, 0.057mmol) was added thereto and stirred at room temperature for 2 hours. Then, the reaction liquid was filtered, and the obtained solid material was washed with diethyl ether to obtain a title compound (21.6mg, 91%). The characteristic value of the compound is shown below.
¹H-NMR (300 MHz, DMSO): δ 3.87-3.89 (m, 4H), 3.98-4.01 (m, 4H), 6.49 (s, 1H), 7.43-7.57 (m, 2H), 7.88 (d, 2H, J=6.2 Hz), 8.04 (d, 1H, J=7.6 Hz), 8.06 (s, 1H), 8.34 (s, 1H), 8.57 (d, 2H, J=6.2 Hz), 8.59 (s, 1H), 8.71 (d, 1H, J=8.2 Hz), 10.99 (s, 1H); MS (ESI) m/z 456 (M+H)⁺.

### Example 12: Synthesis of N-(2,3-dimethyl-1H-indol-5-yl-methylidene)-N'-(8-morpholin-4-yl-2-pyridin-4-yl-imidazo[1,2-b]pyridazin-6-yl)-hydrazine (Compound 12)

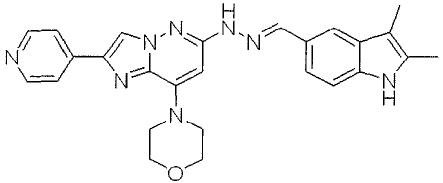

(8-Morpholin-4-yl-2-pyridin-4-yl-imidazo[1,2-b]pyridazin-6-yl)-hydrazine (16.7mg, 0.0536mmol) was dissolved in ethanol (2mL). 2,3-Dimethyl-1H-indole-5-carboxyaldehyde (10.2mg, 0.0590mmol) was added thereto and stirred at room temperature for 2 hours. Then, the reaction liquid was filtered, and the obtained solid material was washed with diethyl ether to obtain a title compound (23.4mg, 94%). The characteristic value of the compound is shown below.
¹H-NMR (300 MHz, DMSO): δ 2.19 (s, 3H), 2.42 (s, 3H), 3.83-3.87 (m, 4H), 4.01-4.04 (m, 4H), 6.49 (s, 1H), 7.02 (t, 1H, J=7.6 Hz), 7.15 (d, 1H, J=7.6 Hz), 7.42 (d, 1H, J=7.6 Hz), 7.89 (d, 2H, J=6.2 Hz), 8.26 (s, 1H), 8.56 (s, 1H), 8.57 (d, 2H, J=6.2 Hz), 10.30 (s, 1H), 10.98 (s, 1H); MS (ESI) m/z 467 (M+H)⁺.

### Example 13: Synthesis of N-(5-methyl-furan-2-yl-methylidene)-N'-(8-morpholin-4-yl-2-pyridin-4-yl-imidazo[1,2-b]pyridazin-6-yl)-hydrazine (Compound 13)

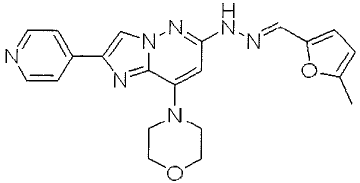

(8-Morpholin-4-yl-2-pyridin-4-yl-imidazo[1,2-b]pyridazin-6-yl)-hydrazine (20.4mg, 0.0656mmol) was dissolved in ethanol (1mL). 5-Methyl-furan-2-carboxyaldehyde (6.5 µL, 0.066mmol) was added thereto and stirred at room temperature for 2 hours. Then, the reaction liquid was filtered, and the obtained solid material was washed with diethyl ether to obtain a title compound (14.5mg, 55%). The characteristic value of the compound is shown below.
¹H-NMR (300 MHz, DMSO): δ 2.32 (s, 3H), 3.81-3.84 (m, 4H), 3.92-3.94 (m, 4H), 6.20 (d, 1H, J=3.1 Hz), 6.28 (s,1H), 6.64 (d, 1H, J=3.1 Hz), 7.84 (s, 1H), 7.86 (d, 2H, J=6.0 Hz), 8.56 (s, 1H), 8.56 (d, 2H, J=6.0 Hz), 10.81 (s, 1H); MS (ESI) m/z 404 (M+H)⁺.

### Example 14: Synthesis of N-(3-isopropyl-benzylidene)-N'-(8-morpholin-4-yl-2-pyridin-4-yl-imidazo[1,2-b]pyridazin-6-yl)-hydrazine (Compound 14)

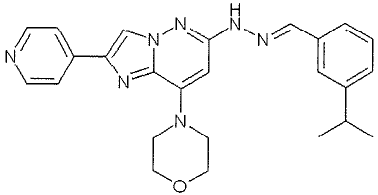

(8-Morpholin-4-yl-2-pyridin-4-yl-imidazo[1,2-b]pyridazin-6-yl)-hydrazine (20.8mg, 0.0668mmol) was dissolved in ethanol (2mL). 3-Isopropyl-benzaldehyde (9.9mg, 0.067mmol) was added thereto and stirred at room temperature overnight. Then, the reaction liquid was filtered, and the obtained solid material was washed with diethyl ether to obtain a title compound (19.4mg, 65%).
¹H-NMR (300 MHz, DMSO): δ 1.22 (d, 6H, J=6.7 Hz), 3.83-3.86 (m, 4H), 3.96-3.98 (m, 4H), 6.45 (s, 1H), 7.22 (d, 1H, J=7.6 Hz), 7.33 (t, 1H, J=7.6 Hz), 7.48 (s, 1H), 7.53 (d, 1H, J=7.6 Hz), 7.87 (d, 2H, J=6.2 Hz), 8.01 (s, 1H), 8.57 (d, 2H, J=6.2 Hz), 8.57 (s, 1H), 10.96 (s, 1H); MS (ESI) m/z 442 (M+H)⁺.

### Example 15: Synthesis of N-(1H-indol-6-yl-methylidene)-N'-(8-morpholin-4-yl-2-pyridin-4-yl-imidazo[1,2-b]pyridazin-6-yl)-hydrazine (Compound 15)

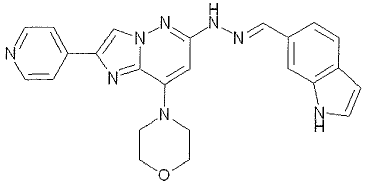

(8-Morpholin-4-yl-2-pyridin-4-yl-imidazo[1,2-b]pyridazin-6-yl)-hydrazine (20.3mg, 0.0652mmol) was dissolved in ethanol/dichloromethane = 1:1 (2mL). 1H-Indole-6-carboxyaldehyde (9.5mg, 0.065mmol) was added thereto and stirred at room temperature overnight. Then, the solvent was removed from the reaction liquid, and the obtained residue was washed with diethyl ether to obtain a title compound (19.4mg, 68%).
¹H-NMR (300 MHz, DMSO): δ 3.85-3.87 (m, 4H), 3.96-3.98 (m, 4H), 6.44 (s, 1H), 6.47 (s, 1H), 7.39-7.45 (m, 2H), 7.54-7.59 (m, 2H), 7.87 (d, 2H, J=6.2 Hz), 8.10 (s, 1H), 8.56-8.58 (m, 3H), 10.76 (s, 1H), 11.21 (s, 1H); MS (ESI) m/z 439 (M+H)⁺.

### Example 16: Synthesis of N-(3-isopropenyl-benzylidene)-N'-(8-morpholin-4-yl-2-pyridin-4-yl-imidazo[1,2-b]-pyridazin-6-yl)-hydrazine (Compound 16)

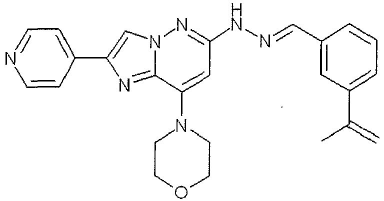

(8-Morpholin-4-yl-2-pyridin-4-yl-imidazo[1,2-b]pyridazin-6-yl)-hydrazine (20.6mg, 0.0662mmol) was dissolved in ethanol (2mL). 3-Isopropenyl-benzaldehyde (9.7mg, 0.066mmol) was added thereto and stirred at room temperature overnight. Then, the reaction liquid was filtered, and the obtained solid material was washed with diethyl ether to obtain a title compound (21.2mg, 78%).
¹H-NMR (300 MHz, DMSO): δ 2.14 (s, 3H), 3.83-3.85 (m, 4H), 3.60-3.78 (m, 4H), 5.14 (s, 1H), 5.48 (s, 1H), 6.48 (s, 1H), 7.39 (t, 1H, J=7.5 Hz), 7.48 (d, 1H, J=7.5 Hz), 7.67 (d, 1H, J=7.5 Hz), 7.71 (s, 1H), 7.87 (d, 2H, J=6.0 Hz), 8.04 (s, 1H), 8.57 (d, 2H, J=6.0 Hz), 8.57 (s, 1H), 11.02 (s, 1H); MS (ESI) m/z 440 (M+H)⁺.

### Example 17: Synthesis of N-(3-acetyl-benzylidene)-N'-(8-morpholin-4-yl-2-pyridin-4-yl-imidazo[1,2-b]pyridazin-6-yl)-hydrazine (Compound 17)

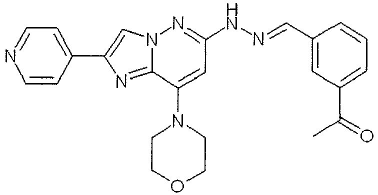

(8-Morpholin-4-yl-2-pyridin-4-yl-imidazo[1,2-b]pyridazin-6-yl)-hydrazine (15.1mg, 0.0485mmol) was dissolved in ethanol (2mL). 3-Acetyl-benzaldehyde (7.2mg, 0.049mmol) was added thereto and stirred at room temperature overnight. Then, the reaction liquid was filtered, and the obtained solid material was washed with diethyl ether to obtain a title compound (16.9mg, 79%).
¹H-NMR (300 MHz, DMSO): δ 2.62 (s, 3H), 3.82-3.86 (m, 4H), 3.97-4.00 (m, 4H), 6.48 (s, 1H), 7.57 (t, 1H, J=7.8 Hz), 7.86-7.92 (m, 3H), 8.01 (d, 1H, J=7.8 Hz), 8.10 (s, 1), 8.14 (s, 1H), 8.57 (d, 1H, J=6.3 Hz), 8.60 (s, 1H), 11.13 (s, 1H); MS (ESI) m/z 442 (M+H)⁺.

### Example 18: Synthesis of N-[3-(1-methyl-pentyl)-benzylidene]-N'-(8-morpholin-4-yl-2-pyridin-4-yl-imidazo[1,2-b]pyridazin-6-yl)-hydrazine (Compound 18)

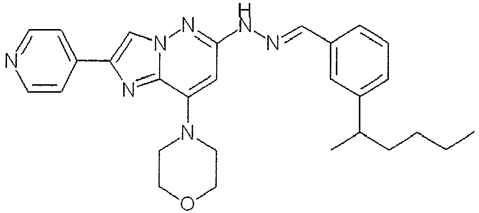

(8-Morpholin-4-yl-2-pyridin-4-yl-imidazo[1,2-b]pyridazin-6-yl)-hydrazine (12.9mg, 0.0414mmol) was dissolved in ethanol (2mL). 3-(1-Methyl-pentyl)-benzaldehyde (9.5mg, 0.065mmol) was added thereto and stirred at room temperature overnight. Then, the solvent was removed from the reaction liquid, and the obtained residue was washed with diethyl ether to obtain a title compound (8.7mg, 45%).
¹H-NMR (300 MHz, DMSO): δ 0.81 (t, 3H, J=7.3 Hz), 1.07-1.28 (m, 4H), 1.20 (d, 3H, J=6.7 Hz), 1.55 (q, 2H, J=7.3 Hz), 2.77 (q, 1H, J=6.7 Hz), 3.83-3.85 (m, 4H), 3.95 (m, 4H), 6.45 (s, 1H), 7.18 (d, 1H, J=7.6 Hz), 7.32 (t, 1H, J=7.6 Hz), 7.43 (s, 1H), 7.53 (d, 1H, J=7.6 Hz), 7.87 (d, 2H, J=6.2 Hz), 8.00 (s, 1H), 8.57 (d, 2H, J=6.2 Hz), 8.57 (s, 1H), 10.96 (s, 1H); MS (ESI) m/z 484 (M+H)⁺.

### Example 19: Synthesis of N-(3-vinyl-benzvlidene)-N-(8-mor-pholin-4-yl-2-pyridin-4-yl-imidazo[1,2-b]pyridazin-6-yl)-hydrazine (Compound 19)

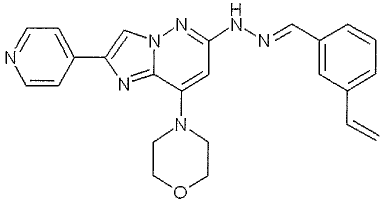

3-Vinylbenzaldehyde (17mg, 0.13mmol) and an acetic acid (5.0 µL) were added to an ethanol solution (2.0mL) of (8-morpholin-4-yl-2-pyridin-4-yl-imidazo[1,2-b]pyridazin-6-yl)-hydrazine (20mg, 0.064mmol), and stirred at room temperature for 3 hours. The precipitated solid material was filtered and washed with ethanol to obtain a title compound (20mg, 72%).
¹H-NMR (300MHz, DMSO) : δ 3.85-3.87 (4H, m), 3.99-4.02 (4H, m), 5.33 (1H, J = 11.4Hz, d), 5.92 (1H, J = 17.4Hz, d), 6.49 (1H, s), 6.80 (1H, J = 10.5, 17.4Hz, dd), 7.41 (1H, J = 7.5Hz, t), 7.45 (1H, J = 8.1Hz, d), 7.66 (1H, J = 8.1Hz, d), 7.71 (1H, s), 7.88 (1H, s), 7.90 (1H, s), 8.05 (1H, s), 8.58-8.59 (3H, m), 11.04 (1H, s) MS(ESI) m/z (M+H)⁺ 426

### Example 20: Synthesis of N-benzofuran-5-ylmethylene-N'-(8-morpholin-4-yl-2-pyridin-4-yl-imidazo[1,2-b]pyridazin-6-yl)-hydrazine (Compound 20)

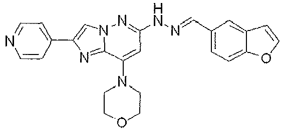

(8-Morpholin-4-yl-2-pyridin-4-yl-imidazo[1,2-b]pyridazin-6-yl)-hydrazine (25.0mg, 0.0803mmol) was dissolved in ethanol (2mL). Benzofuran-5-carboxyaldehyde (11.7mg, 0.0803mmol) was added thereto and stirred at room temperature overnight. Then, the reaction liquid was filtered, and the obtained solid material was washed with diethyl ether to obtain a title compound (24.9mg, 71%).
¹H-NMR (300 MHz, DMSO): δ 3.83-3.87 (m, 4H), 3.97-4.00 (m, 4H), 6.48 (s, 1H), 6.99 (d, 1H, J=1.5 Hz), 7.63 (d, 1H, J=8.5 Hz), 7.73 (dd, 1H, J=1.5, 8.5 Hz), 7.87 (d, 2H, J=6.0 Hz), 7.90 (d, 1H, J=1.5 Hz), 8.13 (s, 1H), 8.57 (d, 2H, J=6.0 Hz), 8.57 (s, 1H), 10.91 (s, 1H); MS (ESI) m/z 440 (M+H)⁺.

### Example 21: Synthesis of N-(1H-indol-2-ylmethylene)-N-(8-morpholin-4-yl-2-pyridin-4-yl-imidazo [1,2-b]pyridazin-6-yl)-hydrazine (Compound 21)

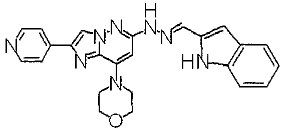

2-Formylindole (19mg, 0.13mmol) and an acetic acid (5.0 µL) were added to an ethanol solution (2.0mL) of (8-morpholin-4-yl-2-pyridin-4-yl-imidazo[1,2-b]pyridazin-6-yl)-hydrazine (20mg, 0.064mmol), and stirred at room temperature for 3 hours. The precipitated solid material was filtered and washed with ethanol to obtain a title compound (20mg, 71%).
¹H-NMR (300MHz, DMSO) : δ 3.87-3.88 (4H, m), 4.04-4.06 (4H, m), 6.63 (1H, s), 6.67 (1H, s), 6.96-7.01 (1H, m), 7.10-7.15 (1H, m), 7.41 (1H, J = 8.1Hz, d), 7.51 (1H, J = 8.1Hz, d), 7.87-7.89 (2H, m), 8.05 (1H, s), 8.55-8.58 (3H, m), 11.00 (1H, s), 11.30 (1H, s); MS(ESI) m/z 439 (M+H)⁺.

### Example 22: Synthesis of N-(3-difluoromethyl-benzylidene)-N-(8-morpholin-4-yl-2-pyridin-4-yl-imidazo[1,2-b]pyridazin-6-yl)-hydrazine (Compound 22)

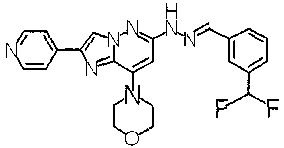

3-Difluoromethylbenzaldehyde (26mg, 0.17mmol) and an acetic acid (5.0 µL) were added to an ethanol solution (2.0mL) of (8-morpholin-4-yl-2-pyridin-4-yl- imidazo[1,2-b]pyridazin-6-yl)-hydrazine (35mg, 0.11mmol) and stirred at room temperature for 3 hours. The precipitated solid material was filtered and washed with ethanol to obtain a title compound (24mg, 48%).
¹H-NMR (300MHz, DMSO) : δ 3.86-3.88 (4H, m), 4.00-4.02 (4H, m), 6.47 (1H, s), 7.10 (1H, J = 56Hz, t), 7.54-7.61 (2H, m), 7.84 (1H, s), 7.89-7.91 (3H, m), 8.10 (1H, s), 8.59-8.62 (3H, m), 11.12 (1H, s); MS(ESI) m/z 450 (M+H)⁺.

### Example 23: Synthesis of N-(3-dimethylamino-benzylidene)-N-(8-morpholin-4-yl-2-pyridin-4-yl-imidazo[1,2-b]pyridazin-6-yl)-hydrazine (Compound 23)

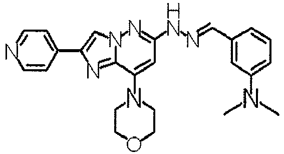

3-Dimethylaminobenzaldehyde (25mg, 0.17mmol) and an acetic acid (5.0 µL) were added to an ethanol solution (2.0mL) of (8-morpholin-4-yl-2-pyridin-4-yl- imidazo[1,2-b]pyridazin-6-yl)-hydrazine (35mg, 0.11mmol), and stirred at room temperature for 3 hours. The precipitated solid material was filtered and washed with ethanol to obtain a title compound (18mg, 36%).
¹H-NMR (300MHz, DMSO) : δ 2.95 (6H, s), 3.85-3.87 (4H, m), 3.97-4.03 (4H, m), 6.49 (1H, s), 6.73 (1H, J = 2.0, 8.4 Hz, dd), 6.96 (1H, J = 2.0Hz, d), 7.04 (1H, J = 7.6Hz, d), 7.23 (1H, J = 7.6Hz, t), 7.89 (1H, J = 2.8Hz, d), 7.89 (1H, J = 6.0Hz, d), 7.98 (1H, s), 8.58-8.60 (3H, m), 10.95 (1H, m); MS(ESI) m/z 443 (M+H)⁺.

### Example 24: Synthesis of N-(5-methyl-1H-pyrrol-2-ylmethylene)-N-(8-morpholin-4-yl-2-pyridin-4-yl-imidazo[12-b]pyridazin-6-yl)-hydrazine (Compound 24)

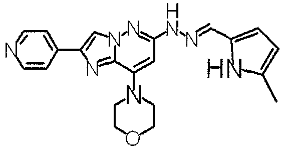

5-Methyl-1H-pyrrole-2-carboxyaldehyde (18mg, 0.17mmol) and an acetic acid (5.0 µL) were added to an ethanol solution (2.0mL) of (8-morpholin-4-yl-2-pyridin-4-yl- imidazo[1,2-b]pyridazin-6-yl)-hydrazine (35mg, 0.11mmol), and stirred at room temperature for 3 hours. The precipitated solid material was filtered and washed with ethanol to obtain a title compound (15mg, 33%).
¹H-NMR (300MHz, DMSO) : δ 2.26 (3H, s), 3.86-3.88 (4H, m), 3.99-4.02 (4H, m), 5.81 (1H, J = 2.4Hz, t), 6.21 (1H, J = 2.4Hz, t), 6.52 (1H, s), 7.81 (1H, s), 7.87 (1H, J = 1.6 Hz, d), 7.89 (1H, J = 1.6Hz, d), 8.51 (1H, S), 8.57-8.59 (2H, m), 10.50 (1H, s), 10.95 (1H, s); MS(ESI) m/z 403 (M+H)⁺.

### Example 25: Synthesis of N-(4-methyl-benzylidene)-N'-(8-morpholin-4-yl-2-pyridin-4-yl-imidazo[1,2-b]pyridazin-6-yl)-hydrazine (Compound 25)

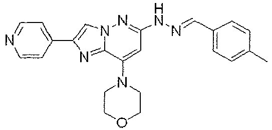

(8-Morpholin-4-yl-2-pyridin-4-yl-imidazo[1,2-b]pyridazin-6-yl)-hydrazine (42.8mg, 0.138mmol) was dissolved in ethanol (4mL). 4-Methyl-benzaldehyde (16.3 µL, 0.138mmol) was added thereto and stirred at room temperature overnight. Then, the reaction liquid was filtered, and the obtained solid material was washed with diethyl ether to obtain a title compound (50.1mg, 88%).
¹H-NMR (400 MHz, DMSO): δ 2.34 (s, 3H), 3.85-3.87 (m, 4H), 3.97-4.00 (m, 4H), 6.47 (s, 1H), 7.24 (d, 2H, J=8.1 Hz), 7.58 (d, 2H, J=8.1 Hz), 7.88 (d, 2H, J=6.1 Hz), 8.01 (s, 1H), 8.58 (s, 1H), 8.58 (d, 2H, J=6.1 Hz), 10.91 (s, 1H); MS (ESI) m/z 414 (M+H)⁺.

### Example 26: Synthesis of N-(4-fluoromethyl-benzylidene)-N'-(8-morpholin-4-yl-2-pyridin-4-yl-imidazo[12-b]pyridazin-6-yl)-hydrazine (Compound 26)

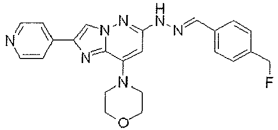

(8-Morpholin-4-yl-2-pyridin-4-yl-imidazo[1,2-b]pyridazin-6-yl)-hydrazine (29.8mg, 0.0958mmol) was dissolved in ethanol (3mL). 4-Fluoromethyl-benzaldehyde (13.2mg, 0.0958mmol) was added thereto and stirred at room temperature overnight. Then, the reaction liquid was filtered, and the obtained solid material was washed with diethyl ether to obtain a title compound (50.1mg, 88%).
¹H-NMR (400 MHz, DMSO): δ 3.85-3.87 (m, 4H), 3.99-4.01 (m, 4H), 5.44 (d, 2H, J=48 Hz), 6.49 (s, 1H), 7.47 (d, 2H, J=7.8 Hz), 7.74 (d, 2H, J=7.8 Hz), 7.89 (d, 2H, J=6.1 Hz), 8.05 (s, 1H), 8.59 (d, 2H, J=6.1 Hz), 8.60 (s, 1H), 11.06 (s, 1H); MS (ESI) m/z 432 (M+H)⁺.

### Example 27: Synthesis of N-(N-methylindoline-6-lymethylene)-N-(8-morpholin-4-yl-2-pyridin-4-yl-imidazo[12-b]pyridazin-6-yl)-hydrazine (Compound 27)

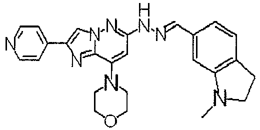

N-Methylindoline-6-carboxyaldehyde (22mg, 0.14mmol) and an acetic acid (5.0 µL) were added to an ethanol solution (2.0mL) of (8-morpholin-4-yl-2-pyridin-4-yl- imidazo[1,2-b]pyridazin-6-yl)-hydrazine (35mg, 0.11mmol), and stirred at room temperature for 3 hours. The precipitated solid material was filtered and washed with ethanol to obtain a title compound (32mg, 63%).
¹H-NMR (300MHz, DMSO) : δ 2.78 (3H, s), 2.90 (2H, J = 8.0Hz, d), 3.28-332 (2H, m), 3.73-3.75 (4H, m), 3.88-3.91 (4H, m), 6.37 (1H, s), 6.74 (1H, s), 6.84 (1H, s), 6.92 (1H, J = 1.2, 7.2Hz, dd), 7.09 (1H, J = 7.2Hz, d), 7.91-7.93 (2H, m), 8.00 (1H, s), 8.66 (1H, J = 2.8Hz, d), 8.66 (1H, J = 6.0Hz, d), 11.18 (1H, s); MS(ESI) m/z 455 (M+H)⁺.

### Example 28: Synthesis of N-(2-methyl-benzylidene)-N'-(8-morpholin-4-yl-2-pyridin-4-yl-imidazo[1,2-b]pyridazin-6-yl)-hydrazine (Compound 28)

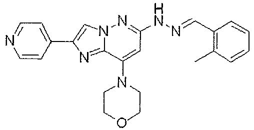

(8-Morpholin-4-yl-2-pyridin-4-yl-imidazo[1,2-b]pyridazin-6-yl)-hydrazine (34.5mg, 0.111mmol) was dissolved in ethanol (3mL). 2-Methyl-benzaldehyde (13.3mg, 0.111mmol) was added thereto and stirred at room temperature overnight. Then, the reaction liquid was filtered, and the obtained solid material was washed with diethyl ether to obtain a title compound (35.7mg, 78%).
¹H-NMR (400 MHz, DMSO): δ 2.45 (s, 3H), 3.84-3.87 (m, 4H), 3.97-3.99 (m, 4H), 6.48 (s, 1H), 7.23-7.24 (m, 3H), 7.82 (m, 1H), 7.85 (d, 2H, J=6.1 Hz), 8.31 (s, 1H), 8.58 (d, 2H, J=6.1 Hz), 8.59 (s, 1H), 10.97 (s, 1H); MS (ESI) m/z 414 (M+H)⁺.

### Example 29: Synthesis of N-(3-methyl-benzylidene)-N'-{8-morpholin-4-yl-2-(1H-pyrazol-4-yl)-imidazo[1,2-b]pyridazin-6-yl}-hydrazine (Compound 29) (Step 1)

### 6-Chloro-2-{1-(4-methoxy-benzyl)-1H-pyrazol-4-yl}-8-morpholin-4-yl-imidazo [1,2-b] pyridazine

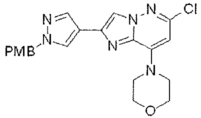

4-Bromo-6-chloropyridazin-3-ylamine (500mg, 2.40mmol) was dissolved in ethanol (20mL). 2-Bromo-1-{1-(4-methoxy-benzyl)-1H-pyrazol-4-yl} -ethanone (1.11g, 3.60mmol) was added thereto and stirred with heating under reflux overnight. Then, the solvent was removed from the reaction liquid, and the obtained solid material was washed with an ethyl acetate. The obtained solid material was dissolved in 1,4-dioxane (10mL), and morpholine (419µL, 4.80mol) was added thereto and stirred at room temperature overnigh. The solvent was removed from the reaction mixture, and the residue was diluted with water and extracted with an ethyl acetate. The extract together with the extraction liquid was dried with anhydrous sodium sulfate, and then the solvent was removed therefrom. The obtained solid material was washed with methanol to obtain a title compound (432mg, 42%).
¹H-NMR (400 MHz, DMSO): δ 3.73 (s, 3H), 3.77-3.78 (m, 4H), 4.07-4.09 (m, 4H), 5.28 (s, 2H), 6.40 (s, 1H), 6.91 (d, 2H, J=8.7 Hz), 7.25 (d, 2H, J=8.7 Hz), 7.89 (d, 1H, J=0.5 Hz), 8.18 (d, 1H, J=0.5 Hz), 8.24 (s, 1H); MS (ESI) m/z 425 (M+H)⁺.

### (Step 2)

### N-(3-methyl-benzylidene)-N'-{8-morpholin-4-yl-2-(1H-pyrazol-4-yl)-imidazo[ 1,2-b] pyridazin-6-yl}-hydrazine (Compound 29)

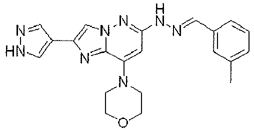

6-Chloro-2-{1-(4-methoxy-benzyl) -1H-pyrazol-4-yl}- 8-morpholin-4-yl -imidazo [1,2-b]pyridazine (100mg, 0.235mmol) was dissolved in N-methylpyrrolidone (2mL). Potassium carbonate (65.0mg, 0.470mmol) and hydrazine monohydrate (114 µL) were added thereto and stirred in a sealed tube at 150°C for 6 hours. Thus obtained reaction liquid was diluted with water and extracted with an ethyl acetate. The extract together with the extraction liquid was dried with anhydrous sodium sulfate, and then the solvent was removed therefrom. The obtained solid material was dissolved in ethanol (4mL), and 3-methylbenzaldehyde (27.7 µL) was added thereto and stirred at room temperature overnight. Then, the reaction liquid was filtered, and the obtained solid material was dissolved in a trifluoroacetic acid (3mL), and stirred in a sealed tube at 100°C for 15 minutes under irradiating microwave. The solvent was removed from the reaction liquid, and the obtained residue was purified with reversed-phase HPLC and dechlorinated to obtain a title compound (14.4mg, 15%).
¹H-NMR (400 MHz, DMSO): δ 2.35 (s, 3H), 3.82-3.85 (m, 4H), 3.94-3.96 (m, 4H), 6.41 (s, 1H), 7.16 (d, 1H, J=7.6 Hz), 7.30 (t, 1H, J=7.6 Hz), 7.45 (s, 1H), 7.50 (d, 1H, J=7.6 Hz), 7.86 (br, 1H), 7.98 (s, 1H), 8.06 (br, 1H), 10.85 (s, 1H), 12.88 (br, 1 H); MS (ESI) m/z 403 (M+H)⁺.

### Example 30: Synthesis of N-(1H-indol-3-ylmethylene)-N'-[2-(6-methyl-pyridin-3-yl)-8-morpholin-4-yl-imidazo[1,2-b]pyridazin-6-yl]-hydrazine (Compound 30)

### (Step 1)

### 6-Chloro-2-(6-methyl-pyridin-3-yl)-8-morpholin-4-yl-imidazo[1,2-b] pyridazine

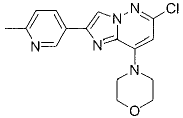

4-Bromo-6-chloropyridazin-3-ylamine (500mg, 2.40mmol) was dissolved in ethanol (20mL). 2-Bromo-1-(6-methyl-pyridin-3-yl)-ethanone hydrobromate (1.06g, 3.60mmol) was added thereto and stirred with heating under reflux overnight. The reaction liquid was cooled down to room temperature, and morpholine (3mL, 36.5mol) was added thereto and stirred at room temperature for 3 hours. The reaction mixture was filtered, and the obtained solid material was diluted with water and extracted with methylene chloride. The extract together with the extraction liquid was dried with anhydrous sodium sulfate, and then the solvent was removed therefrom. The obtained solid material was washed with methanol to obtain a title compound (308mg, 39%).
¹H-NMR (400 MHz, C D Cl₃): δ 2.60 (s, 3H), 3.93 (t, 4H, J=4.8 Hz), 4.10 (t, 4H, J=4.8 Hz), 6.09 (s, 1H), 7.21 (d, 1H, J=8.0 Hz), 8.03 (s, 1H), 8.06 (dd, 1H, J=2.3, 8.0 Hz), 9.00 (d, 1H, J=2.3 Hz); MS (ESI) m/z 330 (M+H)⁺.

### (Step 2)

### N-(1H-indol-3-ylmethylene)-N'-[2-(6-methyl-pyridin-3-yl)-8-morpholin-4-yl-i midazo [1,2-b]pyridazin-6-yl]-hydrazine

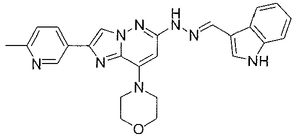

6-Chloro-2-(6-methyl-pyridin-3-yl)-8-morpholin-4-yl-imidazo[1,2-b] pyridazine (170mg, 0.515mmol) was dissolved in N-methylpyrrolidone (2mL). Potassium carbonate (142mg, 1.03mmol) and hydrazine monohydrate (250 µL, 5.15mmol) were added thereto and stirred at 150°C for 2 hours. Thus obtained reaction liquid was diluted with water and extracted with an ethyl acetate. The extract together with the extraction liquid was dried with anhydrous sodium sulfate, and then the solvent was removed therefrom. The obtained residue was dissolved in ethanol (5mL), and 1H-indole-3-carboxyaldehyde (74.6mg, 0.515mmol) was added thereto and stirred at room temperature for 2 hours. Then, the solvent was removed from the reaction liquid, and the obtained residue was purified with NH-silica gel column chromatography to obtain a title compound (20.3mg, 8.7%).
¹H-NMR (400 MHz, DMSO): δ 2.49 (s, 3H), 3.88-3.90 (m,4H), 3.97-3.99 (m, 4H), 6.54 (s, 1H), 7.15-7.22 (m, 2H), 7.30 (d, 1H, J=8.0 Hz), 7.43 (m, 1H), 7.70 (d, 1H, J=2.6 Hz), 8.17 (dd, 1H, J=2.3, 8.0 Hz), 8.22 (m, 1H), 8.25 (s, 1H), 8.39 (s, 1H), 9.01 (d, 1H, J=1.9 Hz), 10.56 (s, 1H), 11.42 (s, 1H); MS (ESI) m/z 453 (M+H)⁺.

### Test Example 1: Evaluation of a cytokine inhibiting action using mouse peritoneal macrophages

1mL of a 3% (w/v) thioglycolate solution was intraperitoneally administered to a mouse (female Balb/c). 5 or 6 days later, the peritoneal cells of the mouse were collected, and the adherent cells were used for evaluation. 100ng/mL of mouse interferon- γ, a 0.05% (v/v) suspension of killed Staphylococcus aureus Cowan I strain (SAC) and a compound of the present invention were added thereto and incubated overnight. After the incubation, the survival rate was measured, and each of IL-12p70 (IL-12p35/p40 complex) and TNF-α was quantified by ELISA using the collected culture supernatant to calculate the 50% production inhibition concentration (IC₅₀) (refer to Table 1; meanwhile, the blanks in the table indicate unmeasured).

From the results shown in the table, it is clarified that the imidazopyridazine compound of the present invention has an excellent inhibiting activity against IL-12 production.

Further, the same measurement by ELISA can also be conducted to IL-12p40 or IL-23, and it is possible to confirm that the imidazopyridazine compound of the present invention has an excellent inhibiting activity against IL-23 production.

**Table 1**

| | IL-12p70 | TNFα |
|---|---|---|
| Compound No. | IC50(nM) | IC50(µM) |
| 1 | 32 | |
| 2 | 36 | |
| 3 | 8.7 | >10 |
| 4 | 3.6 | >10 |
| 5 | 33 | |
| 6 | 92 | |
| 7 | 40 | |
| 8 | 15 | |
| 9 | 76 | |
| 10 | 8.1 | |
| 11 | 30 | |
| 12 | 140 | |
| 13 | 190 | |
| 14 | 130 | |
| 15 | 54 | |
| 16 | 77 | |
| 17 | 37 | |
| 18 | 1100 | |
| 19 | 6.4 | |
| 20 | 9.4 | |
| 21 | 16 | |
| 22 | 40 | |
| 23 | 63 | |
| 24 | 370 | |
| 25 | 44 | |
| 26 | 46 | |
| 27 | 44 | |
| 28 | 76 | |
| 29 | 1.1 | |
| 30 | 5.2 | |

### Test Example 2: Evaluation of a cytokine inhibiting action using mouse whole blood

The blood (EDTA addition) was collected from a mouse (female Balb/c) and used for evaluation. 200ng/mL of mouse interferon- γ, a 1.25% (v/v) suspension of killed Staphylococcus aureus Cowan I strain (SAC) and a compound of the present invention were added to a cell culture medium, diluted to become a quantity equal to the blood and incubated overnight. After the incubation, IL-12p70 (IL-12p35/p40 complex) was quantified by ELISA using the collected culture supernatant to calculate the 50% production inhibition concentration (IC₅₀) of each of Compounds 3, 4, 8, 9, 10 and 11 (refer to Table 2). From the results shown in the table, it is clarified that, in the preferable imidazopyridazine compound of the present invention, the inhibiting activity thereof against IL-12/IL-23 production is not significantly reduced in whole blood and thus, the compound has an excellent activity.

**Table 2: Activity in whole blood**

| | IL-12p70 |
|---|---|
| Compound No. | IC50(nM) |
| 3 | 3000 |
| 4 | 470 |
| 8 | 1500 |
| 9 | 890 |
| 10 | 180 |
| 11 | 1000 |
| 20 | 150 |
| 21 | 340 |
| 22 | 110 |
| 23 | 550 |
| 25 | 63 |
| 26 | 270 |
| 27 | 1200 |
| 28 | 290 |
| 29 | 1400 |
| 30 | 1900 |

## Claims

1. An imidazopyridazine compound of the following formula (I) or pharmaceutically acceptable salts thereof: 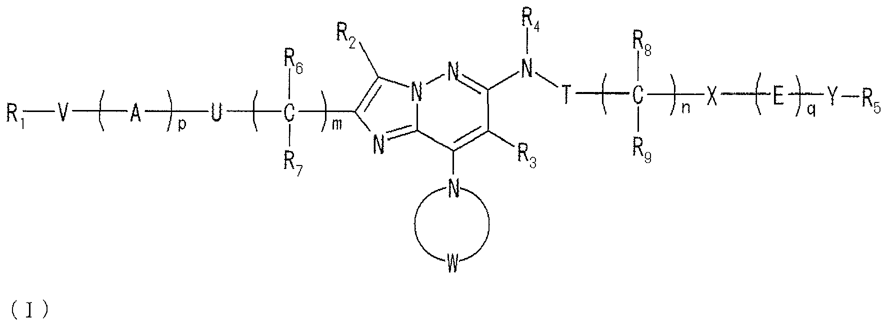
wherein A and E may be same or different from each other and each independently represents an aryl group which may have a substituent(s), a heterocyclic group which may have a substituent(s) or an aliphatic cyclic group which may have a substituent(s);
U, V, X and Y may be same or different from each other and each independently represents a single bond, O, S, S(O), S(O₂), NRa, C(O), C(O)O, C(O)NRa, OC(O), NRaC(O), NRaC(O)NRb, OC(O)NRa, NRaC(O)O, S(O)NRa, S(O₂)NRa, NRaS(O), NRaS(O₂), CRa=CRb, C=C or CRa=N wherein Ra and Rb each independently represents a hydrogen atom or a straight or branched alkyl group having 1 to 3 carbon atoms;
T represents NRcC(RdRe) or N=C(Rd) wherein Rc, Rd and Re each independently represents a hydrogen atom or a straight or branched alkyl group having 1 to 3 carbon atoms;
m and n may be same or different from each other and each independently represents 0, 1 or 2;
p and q may be same or different from each other and each independently represents 0 or 1;
R₁ represents a hydrogen atom, a halogeno group, a hydroxyl group, an alkyl group which may have a substituent(s), a mercapto group, an alkoxy group which may have a substituent(s), an alkylthio group which may have a substituent(s), an alkylsulfonyl group which may have a substituent(s), an acyl group which may have a substituent(s), an acyloxy group which may have a substituent(s), an amino group which may have a substituent(s), a carboxyl group, an alkoxycarbonyl group which may have a substituent(s), a carbamoyl group which may have a substituent(s), a nitro group, a cyano group, a trifluoromethyl group, a sulfonic group, a sulfonamide group which may have a substituent(s), a sulfinamide group which may have a substituent(s), an aliphatic cyclic group which may have a substituent(s), an alkenyl group which may have a substituent(s), an alkynyl group which may have a substituent(s), a benzyloxy group which may have a substituent(s), an aryloxy group which may have a substituent(s), an arylvinyl group which may have a substituent(s), a heteroaryloxy group which may have a substituent(s), an arylamino group which may have a substituent(s), a heteroarylamino group which may have a substituent(s), an arylethynyl group which may have a substituent(s), an aralkyl group which may have a substituent(s), an aryl group which may have a substituent(s) or a heterocyclic group which may have a substituent(s);
R₂ represents a hydrogen atom, a halogeno group or a straight or branched alkyl group having 1 to 3 carbon atoms;
R₃ represents a hydrogen atom or a straight or branched alkyl group having 1 to 3 carbon atoms;
R₄ represents a hydrogen atom or a straight or branched alkyl group having 1 to 3 carbon atoms;
R₅ represents a hydrogen atom, a halogeno group, a hydroxyl group, a boronyl group which may have a substituent(s), an alkyl group which may have a substituent(s), a mercapto group, an alkoxy group which may have a substituent(s), an alkylthio group which may have a substituent(s), an alkylsulfonyl group which may have a substituent(s), an acyl group which may have a substituent(s), an acyloxy group which may have a substituent(s), an amino group which may have a substituent(s), a carboxyl group, an alkoxycarbonyl group which may have a substituent(s), a carbamoyl group which may have a substituent(s), a nitro group, a cyano group, a trifluoromethyl group, a sulfonic group, a sulfonamide group which may have a substituent(s), a sulfinamide group which may have a substituent(s), an aliphatic cyclic group which may have a substituent(s), an alkenyl group which may have a substituent(s), an alkynyl group which may have a substituent(s), a benzyloxy group which may have a substituent(s), an aryloxy group which may have a substituent(s), an arylvinyl group which may have a substituent(s), a heteroaryloxy group which may have a substituent(s), an arylamino group which may have a substituent(s), a heteroarylamino group which may have a substituent(s), an arylethynyl group which may have a substituent(s), an aralkyl group which may have a substituent(s), an aryl group which may have a substituent(s) or a heterocyclic group which may have a substituent(s);
R₆, R₇, R₈ and R₉ may be same or different from each other and each independently represents a hydrogen atom, a halogeno group or a straight or branched alkyl group having 1 to 3 carbon atoms; and
the group of the following (II) having W represents a four- to six-membered cyclic amino group which may have a substituent(s), wherein W represents O, S or NRf (Rf represents a hydrogen atom or a straight or branched alkyl group having 1 to 3 carbon atoms):

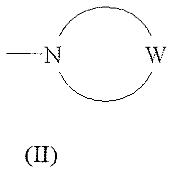

2. The imidazopyridazine compound or pharmaceutically acceptable salts thereof according to claim 1, wherein p is 1.

3. The imidazopyridazine compound or pharmaceutically acceptable salts thereof according to claim 1 or 2, wherein A is an aryl group which may have a substituent(s) or a heterocyclic group which may have a substituent(s).

4. The imidazopyridazine compound or pharmaceutically acceptable salts thereof according to any one of claims 1 to 3, wherein R₁ is a hydrogen atom, a halogeno group, a hydroxyl group, an alkyl group which may have a substituent(s), an alkoxy group which may have a substituent(s), a carboxyl group, an acyl group which may have a substituent(s), an amino group which may have a substituent(s), an alkoxycarbonyl group which may have a substituent(s), an aliphatic cyclic group which may have a substituent(s), a benzyloxy group which may have a substituent(s), an aralkyl group which may have a substituent(s), an aryl group which may have a substituent(s) or a heterocyclic group which may have a substituent(s).

5. The imidazopyridazine compound or pharmaceutically acceptable salts thereof according to any one of claims 1 to 4, wherein q is 1.

6. The imidazopyridazine compound or pharmaceutically acceptable salts thereof according to any one of claims 1 to 5, wherein E is an aryl group which may have a substituent(s) or a heterocyclic group which may have a substituent(s).

7. The imidazopyridazine compound or pharmaceutically acceptable salts thereof according to any one of claims 1 to 6, wherein R₅ is a hydrogen atom, a halogeno group, a hydroxyl group, a boronyl group which may have a substituent(s), an alkyl group which may have a substituent(s), a mercapto group, an alkoxy group which may have a substituent(s), an alkylthio group which may have a substituent(s), an alkylsulfonyl group which may have a substituent(s), an acyl group which may have a substituent(s), an acyloxy group which may have a substituent(s), an amino group which may have a substituent(s), a carboxyl group, an alkoxycarbonyl group which may have a substituent(s), a carbamoyl group which may have a substituent(s), a nitro group, a cyano group, a monofluoromethyl group, a difluoromethyl group, a trifluoromethyl group, a sulfonic group, a sulfonamide group which may have a substituent(s), a sulfinamide group which may have a substituent(s), an alkenyl group which may have a substituent(s) or an alkynyl group which may have a substituent(s).

8. The imidazopyridazine compound or pharmaceutically acceptable salts thereof according to any one of claims 1 to 7, wherein U, V, X and Y may be same or different from each other and each independently represents a single bond, O, S, S(O), S(O₂), NRa, C(O), C(O)O, CRa=CRb or C≡C wherein Ra and Rb each independently represents a hydrogen atom or a straight or branched alkyl group having 1 to 3 carbon atoms; and
T is N=C(Rd) wherein Rd represents a hydrogen atom or a straight or branched alkyl group having 1 to 3 carbon atoms.

9. The imidazopyridazine compound or pharmaceutically acceptable salts thereof according to any one of claims 1 to 8, wherein the group (II) is a 4-morpholinyl group which may have a substituent(s):

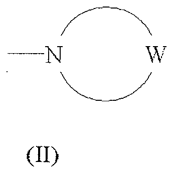

10. The imidazopyridazine compound or pharmaceutically acceptable salts thereof according to any one of claims 1 to 9, wherein n is 0 and m is 0.

11. A pharmaceutical composition comprising the imidazopyridazine compound or pharmaceutically acceptable salts thereof according to any one of claims 1 to 10.

12. A therapeutic or preventive agent for inflammatory diseases comprising the imidazopyridazine compound or pharmaceutically acceptable salts thereof according to any one of claims 1 to 10.

13. A therapeutic or preventive agent for diseases related to IL-12/IL-23 excessive production comprising the imidazopyridazine compound or pharmaceutically acceptable salts thereof according to any one of claims 1 to 10.

14. The therapeutic or preventive agent for diseases related to IL-12/IL-23 excessive production according to claim 13, wherein the disease related to IL-12/IL-23 excessive production is selected from the group consisting of multiple sclerosis, systemic sclerosis, sepsis, myasthenia gravis, autoimmune neurological disease, Guillain-Barre syndrome, autoimmune uveitides, autoimmune hemolytic anemia, pernicious anemia, autoimmune thrombocytopenia, temporal arteritis, antiphospholipid syndrome, vasculitis, Wegener's granulomatosis, Behcet's disease, psoriasis, psoriatic arthritis, herpetic dermatitis, pemphigus vulgaris, vitiligo, Crohn's disease, ulcerative colitis, interstitial fibroid lung, myelofibrosis, hepatic fibrosis, myocarditis, autoimmune thyroid disease, primary biliary cirrhosis, autoimmune hepatitis, immune-mediated diabetes mellitus, autoimmune oophoritis and orchitis, autoimmune adrenalitis, rheumatoid arthritis, juvenile rheumatoid arthritis, systemic lupus erythematosus, scleroderma, polymyositis, dermatomyositis, spondyloarthropathy, ankylosing spondylitis, Sjögren's syndrome and graft versus host disease.

15. The therapeutic or preventive agent for diseases related to IL-12/IL-23 excessive production according to claim 14, wherein the disease related to IL-12/IL-23 excessive production is selected from the group consisting of Crohn's disease, ulcerative colitis, multiple sclerosis, psoriasis, psoriatic arthritis, primary biliary cirrhosis, autoimmune uveitides and rheumatoid arthritis.
